# EUROPÄISCHE PATENTSCHRIFT

(11) **EP 2 091 925 B1**
(45) Veröffentlichungstag und Bekanntmachung des Hinweises auf die Patenterteilung: **06.04.2011**
(21) Anmeldenummer: 07822171.0
(22) Anmeldetag: 03.11.2007
(51) Int. Cl.: C07D 239/94, A61K 31/505, A61P 35/00

(54) **BICYCLISCHE HETEROCYCLEN, DIESE VERBINDUNGEN ENTHALTENDE ARZNEIMITTEL, DEREN VERWENDUNG UND VERFAHREN ZU IHRER HERSTELLUNG**
BICYCLIC HETEROCYCLES, MEDICAMENTS CONTAINING SAID COMPOUNDS, USE THEREOF, AND METHOD FOR PRODUCTION OF SAME
HÉTÉROCYCLES BICYCLIQUES, MÉDICAMENTS CONTENANT CES COMPOSÉS, UTILISATION ET PROCÉDÉ DE PRODUCTION DE CES COMPOSÉS

(30) Priorität: 10.11.2006 EP 06123820
(43) Veröffentlichungstag der Anmeldung: 26.08.2009
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: HIMMELSBACH, Frank, 88441 Mittelbiberach (DE); JUNG, Birgit, 88471 Laupheim (DE)
(74) Vertreter: Hammann, Heinz
(86) Internationale Anmeldenummer: PCT/EP2007/061842
(87) Internationale Veröffentlichungsnummer: WO 2008/055854

(56) Entgegenhaltungen:
- WO-A-2005/028469
- WO-A-2005/028470

## Beschreibung

Gegenstand der vorliegenden Erfindung sind bicyclische Heterocyclen der allgemeinen Formel 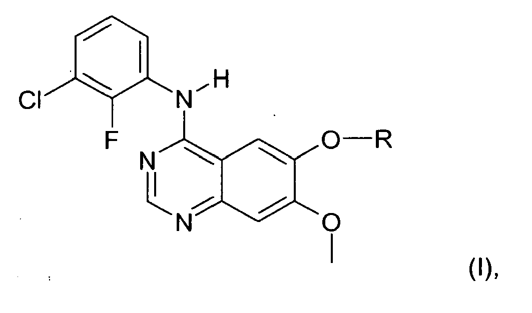 deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

WO-A-2005/028469 und WO-A-2005/028470 offenbaren Quinazolinderivate als Inhibitoren von Tyrosinkinasen bzw. zur Behandlung von Tumorekrankungen.

In der obigen allgemeinen Formel (I) bedeutet
R ein Rest ausgewählt aus der Gruppe bestehend aus
cis-4-Amino-cyclohexyl, trans-4-Amino-cyclohexyl,
cis-4-Methylamino-cyclohexyl, trans-4-Methylamino-cyclohexyl,
cis-4-(Methoxycarbonylamino)-cyclohexyl, trans-4-(Methoxycarbonylamino)-cyclohexyl,
cis-4-(N-Methoxycarbonyl-N-methyl-amino)-cyclohexyl, trans-4-(N-Methoxycarbonyl-N-methyl-amino)-cyclohexyl,
cis-4-(Ethyloxycarbonylamino)-cyclohexyl, trans-4-(Ethyloxycarbonylamino)-cyclohexyl,
cis-4-(N-Ethyloxycarbonyl-N-methyl-amino)-cyclohexyl, trans-4-(N-Ethyloxycarbonyl-N-methyl-amino)-cyclohexyl,
cis-4-(tert.-Butoxycarbonylamino)-cyclohexyl, trans-4-(tert.-Butoxycarbonylamino)-cyclohexyl,
cis-4-(N-tert.-Butoxycarbonyl-N-methyl-amino)-cyclohexyl, trans-4-(N-tert.-Butoxycarbonyl-N-methyl-amino)-cyclohexyl,
cis-4-(Acetylamino)-cyclohexyl, trans-4-(Acetylamino)-cyclohexyl,
cis-4-(N-Acetyl-N-methyl-amino)-cyclohexyl, trans-4-(N-Acetyl-N-methyl-amino)-cyclohexyl,
cis-4-(Methoxyacetyl-amino)-cyclohexyl, trans-4-(Methoxyacetyl-amino)-cyclohexyl,
cis-4-(N-Methoxyacetyl-N-methyl-amino)-cyclohexyl, trans-4-(N-Methoxyacetyl-N-methyl-amino)-cyclohexyl,
cis-4-(Dimethylaminocarbonyl-amino)-cyclohexyl, trans-4-(Dimethylaminocarbonyl-amino)-cyclohexyl,
cis-4-(N-Dimethylaminocarbonyl-N-methyl-amino)-cyclohexyl, trans-4-(N-Dimethylaminocarbonyl-N-methyl-amino)-cyclohexyl,
cis-4-(Morpholinocarbonyl-amino)-cyclohexyl, trans-4-(Morpholinocarbonyl-amino)-cyclohexyl,
cis-4-(N-Morpholinocarbonyl-N-methyl-amino)-cyclohexyl, trans-4-(N-Morpholinocarbonyl-N-methyl-amino)-cyclohexyl,
cis-4-(Piperazin-1--ylcarbonyl-amino)-cyclohexyl, trans-4-(Piperazin-1-ylcarbonyl-amino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl,
cis-4-[(4-Methyl-piperazin-1-ylcarbonyl)-amino]-cyclohexyl, trans-4-[(4-Methylpiperazin-1-ylcarbonyl)-amino]-cyclohexyl,
cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl,
cis-4-(Methansulfonylamino)-cyclohexyl, trans-4-(Methansulfonylamino)-cyclohexyl,
cis-4-(N-Methansulfonyl-N-methyl-amino)-cyclohexyl, trans-4-(N-Methansulfonyl-N-methyl-amino)-cyclohexyl,
cis-4-Phthalimido-cyclohexyl und trans-4-Phthalimido-cyclohexyl,
gegebenenfalls in Form ihrer Tautomeren und ihrer Gemische, sowie gegebenenfalls ihrer pharmakologisch unbedenklichen Säureadditionssalze, Solvate und Hydrate.

Bevorzugt sind Verbindungen, in denen
R ein Rest ausgewählt aus der Gruppe bestehend aus
cis-4-(Methoxycarbonylamino)-cyclohexyl, trans-4-(Methoxycarbonylamino)-cyclohexyl,
cis-4-(N-Methoxycarbonyl-N-methyl-amino)-cyclohexyl-, trans-4-(N-Methoxycarbonyl-N-methyl-amino)-cyclohexyl,
cis-4-(Ethyloxycarbonylamino)-cyclohexyl, trans-4-(Ethyloxycarbonylamino)-cyclohexyl,
cis-4-(N-Ethyloxycarbonyl-N-methyl-amino)-cyclohexyl, trans-4-(N-Ethyloxycarbonyl-N-methyl-amino)-cyclohexyl,
cis-4-(tert.-Butoxycarbonylamino)-cyclohexyl, trans-4-(tert.-Butoxycarbonylamino)-cyclohexyl,
cis-4-(N-tert.-Butoxycarbonyl-N-methyl-amino)-cyclohexyl, trans-4-(N-tert.-Butoxycarbonyl-N-methyl-amino)-cyclohexyl,
cis-4-(Methoxyacetyl-amino)-cyclohexyl, trans-4-(Methoxyacetyl-amino)-cyclohexyl,
cis-4-(N-Methoxyacetyl-N-methyl-amino)-cyclohexyl, trans-4-(N-Methoxyacetyl-N-methyl-amino)-cyclohexyl,
cis-4-(Dimethylaminocarbonyl-amino)-cyclohexyl, trans-4-(Dimethylaminocarbonyl-amino)-cyclohexyl,
cis-4-(N-Dimethylaminocarbonyl-N-methyl-amino)-cyclohexyl, trans-4-(N-Dimethylaminocarbonyl-N-methyl-amino)-cyclohexyl,
cis-4-(Morpholinocarbonyl-amino)-cyclohexyl, trans-4-(Morpholinocarbonyl-amino)-cyclohexyl,
cis-4-(N-Morpholinocarbonyl-N-methyl-amino)-cyclohexyl, trans-4-(N-Morpholinocarbonyl-N-methyl-amino)-cyclohexyl,
cis-4-(Piperazin-1-ylcarbonyl-amino)-cyclohexyl, trans-4-(Piperazin-1-ylcarbonyl-amino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl,
cis-4-[(4-Methyl-piperazin-1-ylcarbonyl)-amino]-cyclohexyl, trans-4-[(4-Methylpiperazin-1-ylcarbonyl)-amino]-cyclohexyl,
cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl,
cis-4-(Methansulfonylamino)-cyclohexyl, trans-4-(Methansulfonylamino)-cyclohexyl,
cis-4-(N-Methansulfonyl-N-methyl-amino)-cyclohexyl, trans-4-(N-Methansulfonyl-N-methyl-amino)-cyclohexyl, cis-4-Phthalimido-cyclohexyl und trans-4-Phthalimidocyclohexyl, bedeutet.

Die Verbindungen der allgemeinen Formel (I) lassen sich beispielsweise nach folgenden Verfahren herstellen:
a) Umsetzung einer Verbindung der Formel 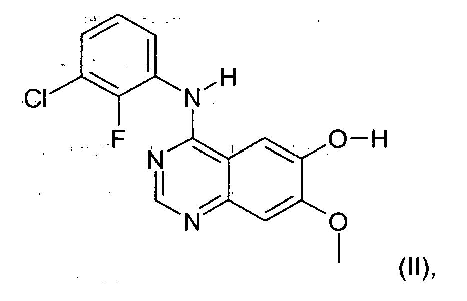 mit einer Verbindung der allgemeinen Formel

   Z¹ - R (III),

   in der
   R wie eingangs erwähnt definiert ist und Z¹ eine Austrittsgruppe wie ein Halogenatom, z.B. ein Chlor- oder Bromatom, eine Sulfonyloxygruppe wie eine Methansulfonyloxy- oder p-Toluolsulfonyloxygruppe oder eine Hydroxygruppe darstellt.
   Die Umsetzung erfolgt zweckmäßigerweise in einem Lösungsmittel wie Ethanol, Isopropanol, Acetonitril, Toluol, Tetrahydrofuran, Dioxan, Dimethylformamid, Dimethylsulfoxid oder N-Methylpyrrolidinon, gegebenfalls in Gegenwart einer Base wie Kaliumcarbonat oder N-Ethyl-diisopropylamin, bei Temperaturen im Bereich von 20°C bis 160°C, vorzugsweise bei Temperaturen im Bereich von 80°C bis 140°C. Mit einer Verbindung der allgemeinen Formel (III), in der Z¹ eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, Dioxan, Toluol oder Ethylenglycoldiethylether bei Temperaturen im Bereich von -50 bis 150°C, vorzugsweise jedoch bei Temperaturen im Bereich von -20 bis 80°C, durchgeführt.
b) Umsetzung einer Verbindung der allgemeinen Formel (IV) 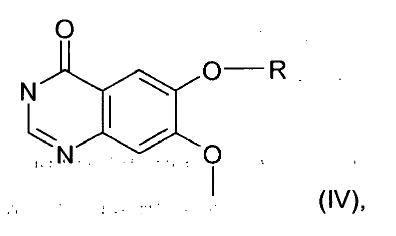 in der R wie eingangs erwähnt definiert ist, mit einem Halogenierungsmittel, beispielsweise einem Säurehalogenid wie Thionylchlorid, Thionylbromid, Phosphortrichlorid, Phosphorpentachlorid oder Phosphoroxychlorid zu einer Zwischenverbindung der allgemeinen Formel (V), 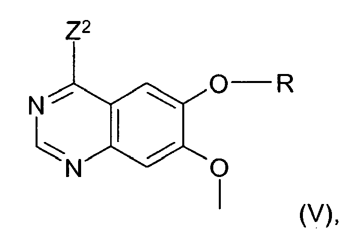 in der R wie eingangs erwähnt definiert ist und Z² ein Halogenatom wie ein Chlor- oder Bromatom darstellt,
   und anschließender Umsetzung mit 3-Chlor-2-fluor-anilin oder dessen Salzen.
   Die Umsetzung mit dem Halogenierungsmittel wird gegebenenfalls in einem Lösungsmittel wie Methylenchlorid, Chloroform, Acetonitril oder Toluol und gegebenfalls in Gegenwart einer Base wie N,N-Diethylanilin oder N-Ethyl-diisopropylamin bei Temperaturen im Bereich von 20°C bis 160°C, vorzugsweise von 40°C bis 120°C durchgeführt. Vorzugsweise wird die Reaktion jedoch mit Thionylchlorid und katalytischen Mengen an Dimethylformamid bei der Siedetemperatur des Reaktionsgemisches durchgeführt.
   Die Umsetzung der Verbindung der allgemeinen Formel (V) mit 3-Chlor-2-fluor-anilin oder dessen Salzen erfolgt zweckmäßigerweise in einem Lösungsmittel wie Ethanol, Isopropanol, Acetonitril, Dioxan oder Dimethylformamid, gegebenfalls in Gegenwart einer Base wie Kaliumcarbonat oder N-Ethyl-diisopropylamin, bei Temperaturen im Bereich von 20°C und 160°C, vorzugsweise von 60°C bis 120°C. Vorzugsweise wird die Reaktion jedoch in Isopropanol bei der Siedetemperatur des Reaktionsgemisches durchgeführt.
c) Zur Herstellung von Verbindungen der allgemeinen Formel (I), in der R ein Rest ausgewählt aus der Gruppe bestehend aus cis-4-(Methoxycarbonylamino)-cyclohexyl, trans-4-(Methoxycarbonylamino)-cyclohexyl, cis-4-(N-Methoxycarbonyl-N-methyl-amino)-cyclohexyl-, trans-4-(N-Methoxycarbonyl-N-methyl-amino)-cyclohexyl, cis-4-(Ethyloxycarbonylamino)-cyclohexyl, trans-4-(Ethyloxycarbonylamino)-cyclohexyl, cis-4-(N-Ethyloxycarbonyl-N-methyl-amino)-cyclohexyl, trans-4-(N-Ethyloxycarbonyl-N-methyl-amino)-cyclohexyl, cis-4-(tert.-Butoxycarbonylamino)-cyclohexyl, trans-4-(tert.-Butoxycarbonylamino)-cyclohexyl, cis-4-(N-tert.-Butoxycarbonyl-N-methyl-amino)-cyclohexyl, trans-4-(N-tert.-Butoxycarbonyl-N-methyl-amino)-cyclohexyl, cis-4-(Acetylamino)-cyclohexyl, trans-4-(Acetylamino)-cyclohexyl,cis-4-(N-Acetyl-N-methyl-amino)-cyclohexyl, trans-4-(N-Acetyl-N-methyl-amino)-cyclohexyl, cis-4-(Methoxyacetyl-amino)-cyclohexyl, trans-4-(Methoxyacetyl-amino)-cyclohexyl,cis-4-(N-Methoxyacetyl-N-methyl-amino)-cyclohexyl, trans-4-(N-Methoxyacetyl-N-methyl-amino)-cyclohexyl, cis-4-(Dimethylaminocarbonyl-amino)-cyclohexyl, trans-4-(Dimethylaminocarbonyl-amino)-cyclohexyl,cis-4-(N-Dimethylaminocarbonyl-N-methyl-amino)-cyclohexyl, trans-4-(N-Dimethylaminocarbonyl-N-methyl-amino)-cyclohexyl, cis-4-(Morpholinocarbonyl-amino)-cyclohexyl, trans-4-(Morpholinocarbonyl-amino)-cyclohexyl,cis-4-(N-Morpholinocarbonyl-N-methyl-amino)-cyclohexyl, trans-4-(N-Morpholinocarbonyl-N-methyl-amino)-cyclohexyl, cis-4-(Piperazin-1-ylcarbonyl-amino)-cyclohexyl, trans-4-(Piperazin-1-ylcarbonyl-amino)-cyclohexyl, cis-4-(N-Piperazin-1-ylcarbonyl-N-methyl-amino)-cyclohexyl, trans-4-(N-Piperazin-1-ylcarbonyl-N-methyl-amino)-cyclohexyl, cis-4-[(4-Methylpiperazin-1-ylcarbonyl)-amino]-cyclohexyl, trans-4-[(4-Methyl-piperazin-1-ylcarbonyl)-amino]-cyclohexyl, cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl, cis-4-(Methansulfonylamino)-cyclohexyl, trans-4-(Methansulfonylamino)-cyclohexyl, cis-4-(N-Methansulfonyl-N-methyl-amino)-cyclohexyl und trans-4-(N-Methansulfonyl-N-methyl-amino)-cyclohexyl bedeutet,
   Umsetzung einer Verbindung der allgemeinen Formel (VI) 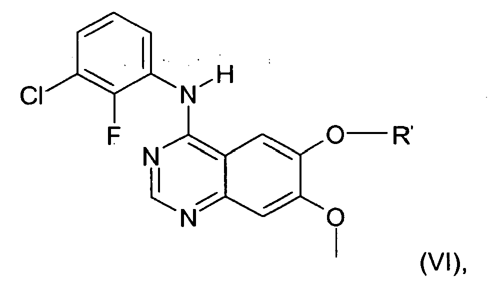 in der R eine cis-4-Amino-cyclohex-1-yl-, trans-4-Amino-cyclohex-1-yl-, cis-4-(Methylamino)-cyclohex-1-yl- oder trans-4-(Methylamino)-cyclohex-1-yl-Gruppe darstellt,
   mit einem entsprechenden Acylierungsmittel wie Chlorameiserisäure-methylester, Chlorameisensäure-ethylester, Pyrokohlensäure-dimethylester, Pyrokohlensäurediethylester, Acetanhydrid, Methoxyacetylchlorid, Dimethylcarbamoylchlorid, Morpholin-4-carbonylchlorid, 4-Methyl-piperazin-1-yl-carbonylchlorid, 4-(tert-Butyloxycarbonyl)-piperazin-1-yl-carbonylchlorid oder Methansulfonylchlorid.
   Die Umsetzung erfolgt zweckmäßigerweise in einem Lösungsmittel wie Methylenchlorid, Acetonitril, Toluol, Tetrahydrofuran, Dioxan, Dimethylformamid, Dimethylsulfoxid oder N-Methylpyrrolidinon, vorzugsweise in Tetrahydrofuran oder Dioxan, gegebenfalls in Gegenwart einer Base wie Kaliumcarbonat, Natronlauge oder N-Ethyl-diisopropylamin, bei Temperaturen im Bereich von -20°C bis 80°C, vorzugsweise von 0°C bis 40°C.
d) Zur Herstellung von Verbindungen der allgemeinen Formel (I), in der R eine cis-4-Phthalimido-cyclohex-1-yl- oder trans-4-Phthalimido-cyclohex-1-yl-Gruppe bedeutet, Umsetzung einer Verbindung der allgemeinen Formel (VII) 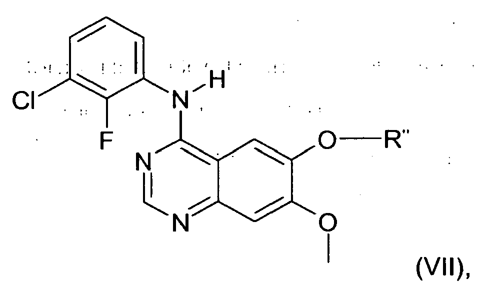 in der R" eine cis-4-Amino-cyclohexyl- oder trans-4-Amino-cyclohexyl-Gruppe darstellt,
   mit Phthalsäureanhydrid oder einem anderen reaktiven Derivat der Phthalsäure.

Die Umsetzung erfolgt zweckmäßigerweise in einem Lösungsmittel wie Essigsäure, Acetonitril, Toluol, Tetrahydrofuran, Dioxan, Dimethylformamid, Dimethylsulfoxid oder N-Methylpyrrolidinon, vorzugsweise in Essigsäure, gegebenfalls in Gegenwart einer Base wie Kaliumcarbonat oder N-Ethyl-diisopropylamin, in einem Temperaturbereich von 60°C bis 160°C, vorzugsweise von 80°C bis 120°C.
Vorzugsweise wird die Reaktion jedoch in Essigsäure bei Temperaturen zwischen 80°C bis 120°C durchgeführt.

Bei den vorstehend beschriebenen Umsetzungen können gegebenenfalls vorhandene reaktive Gruppen wie Amino-, Alkylamino- oder Iminogruppen während der Umsetzung durch übliche Schutzgruppen geschützt werden, welche nach der Umsetzung wieder abgespalten werden.

Als Schutzreste für eine Amino-, Alkylamino- oder Iminogruppe kommen beispielsweise die Formyl-, Acetyl-, Trifluoracetyl-, Methoxycarbonyl, Ethoxycarbonyl- , tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol, Ethanol, Isopropanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung eines Methoxycarbonyl- oder Ethoxycarbonylrestes erfolgt vorzugsweise durch Behandlung mit Natronlauge, gegebenenfalls in Gegenwart eines Lösungsmittels wie Tetrahydrofuran oder Methanol bei Temperaturen zwischen 0 und 50°C.

Ferner können die erhaltenen Verbindungen der allgemeinen Formel I, wie bereits eingangs erwähnt wurde, in ihre Diastereomeren aufgetrennt werden. So können beispielsweise cis-/trans-Gemische durch Chromatographie oder Kristallisation in ihre cis- und trans-Isomere aufgetrennt werden.

Desweiteren können die erhaltenen Verbindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln (II) bis (VII) sind teilweise literaturbekannt (z.B. aus WO 03/82290 oder WO 03/082831) oder können nach an sich literaturbekannten Verfahren (siehe Beispiele I bis X), gegebenenfalls unter zusätzlicher Einführung von Schutzresten, erhalten werden.

Die erfindungsgemäßen Verbindungen der allgemeinen Formel (I) und ihre physiologisch verträglichen Salze weisen wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen werden wie folgt geprüft:

Die Hemmung der humanen EGF-Rezeptorkinase wurde mit Hilfe der cytoplasmatischen Tyrosinkinase-Domäne (Methionin 664 bis Alanin 1186 basierend auf der in Nature 309 (1984), 418 publizierten Sequenz) bestimmt. Hierzu wurde das Protein in Sf9 Insektenzellen als GST-Fusionsprotein unter Verwendung des Baculovirus-Expressionssystems exprimiert.

Die Messung der Enzymaktivität wurde in Gegenwart oder Abwesenheit der Testverbindungen in seriellen Verdünnungen durchgeführt. Das Polymer pEY (4:1) von SIGMA wurde als Substrat verwendet. Biotinyliertes pEY (bio-pEY) wurde als Tracer-Substrat zugesetzt. Jede 100 µl Reaktionslösung enthielt 10 µl des Inhibitors in 50% DMSO, 20 µl der Substrat-Lösung (200 mM HEPES pH 7.4, 50 mM Magnesiumacetat, 2.5 mg/ml poly(EY), 5 µg/ml bio-pEY) und 20 µl Enzympräparation. Die Enzymreaktion wurde durch Zugabe von 50µl einer 100 µM ATP Lösung in 10 mM Magnesiumchlorid gestartet. Die Verdünnung der Enzympräparation wurde so eingestellt, daß der Phosphat-Einbau in das bio-pEY hinsichtlich Zeit und Enzymmenge linear war. Die Enzympräparation wurde in 20 mM HEPES pH 7.4, 1 mM EDTA, 130 mM Kochsalz, 0.05% Triton X-100, 1 mM DTT und 10% Glycerin verdünnt.

Die Enzymassays wurden bei Raumtemperatur über einen Zeitraum von 30 Minuten ausgeführt und durch Zugabe von 50 µl einer Stopplösung (250 mM EDTA in 20 mM HEPES pH 7.4) beendet. 100 µl wurden auf eine Streptavidin-beschichtete Mikrotiterplatte gebracht und 60 Minuten bei Raumtemperatur inkubiert. Danach wurde die Platte mit 200 µl einer Waschlösung (50 mM Tris, 0.05% Tween 20) gewaschen. Nach Zugabe von 100 µl eines HRPO-gelabelten anti-PY Antikörpers (PY20H Anti-PTyr:HRP von Transduction Laboratories, 250 ng/ml) wurde 60 Minuten inkubiert. Danach wurde die Mikrotiterplatte dreimal mit je 200 µl Waschlösung gewaschen. Die Proben wurden dann mit 100 µl einer TMB-Peroxidase-Lösung (A:B = 1:1, Kirkegaard Perry Laboratories) versetzt. Nach 10 Minuten wurde die Reaktion gestoppt. Die Extinktion wurde bei OD₄₅₀nm mit einem ELISA-Leser gemessen. Alle Datenpunkte wurden als Triplikate bestimmt.

Die Daten wurden mittels einer iterativen Rechnung unter Verwendung eines Analysenprogrammes für sigmoidale Kurven (Graph Pad Prism Version 3.0) mit variabler Hill-Steigung angepaßt. Alle freigegebenen Iterationsdaten wiesen einen Korrelationskoeffizienten von über 0.9 auf und die Ober- und Unterwerte der Kurven zeigten eine Spreizung von mindestens einem Faktor von 5. Aus den Kurven wurde die Wirkstoffkonzentration abgeleitet, die die Aktivität der EGF-Rezeptorkinase zu 50% hemmt (IC₅₀). Die erfindungsgemäßen Verbindungen weisen IC₅₀-Werte von unter 100 µM auf. Vorzugsweise weisen die erfindungsgemäßen Verbindungen IC₅₀-Werte von unter 1 µM auf.

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie akute Bronchitis, chronische Bronchitis, chronisch obstruktive Bronchitis (COPD), Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen, sowie akute und chronische Erkrankungen der Nasenschleimhaut und Nasennebenhöhlen, wie akute und chronische Rhinitis, Sinusitis sowie Nasenpolypen.

Die Verbindungen sind auch geeignet für die Behandlung entzündlicher Erkrankungen des Magen-Darm-Traktes und der Gallengänge und -blase, die mit einer gestörten Aktivität der Tyrosinkinasen einhergehen, wie sie z.B. bei akuten oder chronisch entzündlichen Veränderungen zu finden sind, wie Cholezystitis, M. Crohn, Colitis ulcerosa, und Geschwüren oder Polyposis im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome,

desweiteren zur Behandlung von entzündlichen Erkrankungen der Gelenke, wie rheumatoider Arthritis, von entzündlichen Erkrankungen der Haut, der Augen, bei entzündlichen Pseudopolypen, bei Colitis cystica profunda oder bei Pneumatosis cystoides intestinales. Die Verbindungen kommen auch zur Behandlung von ZNS- und Rückenmarksverletzungen in Betracht.

Als bevorzugte Anwendungsgebiete seien entzündliche Erkrankungen der Atemwegsorgane oder des Darmes genannt, wie chronische Bronchitis (COPD), chronische Sinusitis, Asthma, M. Crohn, Colitis ulcerosa oder Polyposis des Darmes. Besonders bevorzugte Anwendungsgebiete sind entzündliche Erkrankungen der Atemwege oder der Lunge wie chronische Bronchitis (COPD) oder Asthma, oder der Nasenschleimhaut und der Nebenhöhlenschleimhaut sowie Nasenpolypen.

Außerdem können die Verbindungen der allgemeinen Formel (I) und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH), inflammatorischer Prozesse, Erkrankungen des Immunsystems, Hyperproliferation hämatopoetischer Zellen, der Behandlung von Nasenpolypen, etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.
Für die Behandlung von Atemwegserkrankungen können diese Verbindungen allein oder in Kombination mit anderen Atemwegstherapeutika, wie z.B. sekretolytisch (z.B. Ambroxol, N-Acetylcystein), broncholytisch (z.B. Tiotropium oder Ipratropium oder Fenoterol, Salmeterol, Salbutamol) und/oder entzündungshemmend (z.B. Theophylline oder Glucocorticoide) wirksamen Substanzen angewendet werden. Gegebenenfalls können die Verbindungen der Formel **1** auch in Kombination mit **W** eingesetzt werden, worin **W** einen pharmakologisch, aktiven Wirkstoff darstellt und (beispielsweise) ausgewählt ist, aus der Gruppe bestehend aus Betamimetika, Anticholinergika, Corticosteroiden, PDE4-Inhibitoren, LTD4-Rezeptor (CysLT1, CysLT2, CysLT3) Antagonisten, EGFR-Hemmern, Dopamin-Agonisten, H1-Antihistaminika, PAF-Antagonisten, SYK-Inhibitoren, PDE3 Inhibitoren, Lipoxin A4 Derivate, FPRL1 Modulatoren, LTB4-Rezeptor (BLT1, BLT2) Antagonisten, Histamin H1 Rezeptor Antagonisten, Histamin H4 Rezeptor Antagonisten, PI3 Kinase Inhibitoren, Inhibitoren von Nicht-Rezeptor Tyrosine Kinasen wie zum Beispiel LYN, LCK, SYK, ZAP-70, FYN, BTK oder ITK, Inhibitoren von MAP Kinasen wie zum Beispiel p38, ERK1, ERK2, JNK1, JNK2, JNK3 oder SAP, Inhibitoren des NF-κB Signalwegs wie zum Beispiel IKK Kinase Inhibitoren, iNOS Inhibitoren, MRP4 Inhibitoren, Leukotriene Biosynthese Inhibitoren wie zum Beispiel 5-Lipoxygenase (5-LO) Inhibitoren, cPLA2 Inhibitoren, Leukotriene A4 Hydrolase Inhibitoren oder FLAP Inhibitoren, Nicht-steroidale antientzündliche Agenzien (NSAIDs), CRTH2 Antagonisten, DP1-Rezeptor Modulatoren, Thromboxane Rezeptor Antagonisten, Chemokine Rezeptor Antagonisten von CCR1, CCR2, CCR2A, CCR2B, CCR4, CCR5, CCR6, CCR7, CCR8, CCR9, CCR10, CCR11, CXCR1, CXCR2, CXCR3, CXCR4, CXCR5, CXCR6, CX3CR1, Neurokinin (NK1, NK2, NK3) Antagonisten, Sphingosine 1-Phosphat Rezeptor Modulatoren, Modulatoren von Adenosine Rezeptoren, Modulatoren von purinergen Rezeptoren wie zum Beispiel P2X7, Histone Deacetylase (HDAC) Aktivatoren, Bradykinin (BK1, BK2) Antagonisten, Modulatoren von Calcitonin Gene Related Peptide (CGRP) wie z.B. CGRP Antagonisten, TACE Inhibitoren, Mucoregulatoren, PPAR gamma Agonisten, Rho Kinase Inhibitoren, interleukin 1-beta converting enzyme (ICE) Inhibitoren, Toll-Like Rezeptor (TLR) Modulatoren, HMG-CoA Reductase Inhibitoren, VLA-4 Antagonisten, ICAM-1 Inhibitoren, SHIP Agonisten, TNFα Antagonisten, GABAa Rezeptor Antagonisten, Immunotherapeutika, Modulatoren des epithelialen Na+-Kanals (ENaC) wie ENaC Inhibitoren, Substanzen gegen Schwellungen der Atemwege und Substanzen gegen Husten.

Weiterhin können zwei- oder dreifach Kombinationen von **W** mit den Verbindungen der Formel 1 kombiniert werden. Beispielhaft genannte Kombinationen von W wären: **W** stellt ein Betamimetikum dar, kombiniert mit einem Anticholinergikum, Corticosteroid, PDE4-Inhibitor, EGFR-Hemmer oder LTD4-Rezeptor Antagonisten, **W** stellt ein Anticholinergikum dar, kombiniert mit einem Betamimetikum, Corticosteroid, PDE4-Inhibitor, EGFR-Hemmer oder LTD4-Rezeptor Antagonisten, **W** stellt ein Corticosteroid dar, kombiniert mit einem PDE4-Inhibitor, EGFR-Hemmer oder Anticholinergikum,
**W** stellt einen PDE4-Inhibitor dar, kombiniert mit einem EGFR-Hemmer oder Anticholinergikum
**W** stellt einen EGFR-Hemmer dar, kombiniert mit Anticholinergika.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
3-(4-{6-[2-Hydroxy-2-(4-hydroxy-3-hydroxymethyl-phenyl)-ethylamino]-hexyloxy}-butyl)-benzyl-sulfonamid
5-[2-(5,6-Diethyl-indan-2-ylamino)-1-hydroxy-ethyl]-8-hydroxy-1H-quinolin-2-on 4-Hydroxy-7-[2-{[2-{[3-(2-phenylethoxy)propyl]sulfonyl}ethyl]-amino}ethyl]-2(3H)-benzoth iazolon
1-(2-Fluor-4-hydroxyphenyl)-2-[4-(1-benzimidazolyl)-2-methyl-2-butylamino]ethanol
1-[3-(4-Methoxybenzyl-amino)-4-hydroxyphenyl]-2-[4-(1-benzimidazolyl)-2-methyl-2-butylamino]ethanol
1-[2H-5-hydroxy-3-oxo-4H-1,4-benzoxazin-8-yl]-2-[3-(4-N,N-dimethylaminophenyl)-2-methyl-2-propylamino]ethanol
1-[2H-5-hydroxy-3-oxo-4H-1,4-benzoxazin-8-yl]-2-[3-(4-methoxyphenyl)-2-methyl-2-propylamino]ethanol
1-[2H-5-hydroxy-3-oxo-4H-1,4-benzoxazin-8-yl]-2-[3-(4-n-butyloxyphenyl)-2-methyl-2-propylamino]ethanol
1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
5-Hydroxy-8-(1-hydroxy-2-isopropylaminobutyl)-2H-1,4-benzoxazin-3-(4H)-on 1-(4-Amino-3-chlor-5-trifluormethylphenyl)-2-tert.-butylamino)ethanol
6-Hydroxy-8-{1-hydroxy-2-[2-(4-methoxy-phenyl)-1,1-dimethyl-ethylamino]-ethyl}-4H-benzo[1,4]oxazin-3-on
6-Hydroxy-8-{1-hydroxy-2-[2-(4-phenoxy-essigsäureethylester)-1,1-dimethyl-ethylamino]-ethyl}-4H-benzo[1,4]oxazin-3-on
6-Hydroxy-8-{1-hydroxy-2-[2-(4-phenoxy-essigsäure)-1,1-dimethyl-ethylamino]-ethyl}-4H-benzo[1,4]oxazin-3-on
8-{2-[1,1-Dimethyl-2-(2,4,6-trimethylphenyl)-ethylamino]-1-hydroxy-ethyl)-6-hydroxy-4H-benzo[1,4]oxazin-3-on
6-Hydroxy-8-{1-hydroxy-2-[2-(4-hydroxy-phenyl)-1,1-dimethyl-ethylamino]-ethyl}-4H-benzo[1,4]oxazin-3-on
6-Hydroxy-8-{1-hydroxy-2-[2-(4-isopropyl-phenyl)-1,1-dimethyl-ethylamino]-ethyl}-4H-benzo[1,4]oxazin-3-<on
8-{2-[2-(4-Ethyl-phenyl)-1,1-dimethyl-ethylamino]-1-hydroxy-ethyl}-6-hydroxy-4H-benzo[1,4]oxazin-3-on
8-{2-[2-(4-Ethoxy-phenyl)-1,1-dimethyl-ethylamino]-1-hydroxy-ethyl}-6-hydroxy-4H-benzo[1,4]oxazin-3-on
4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
8-{2-[2-(3,4-Difluor-phenyl)-1,1-dimethyl-ethylamino]-1-hydroxy-ethyl}-6-hydroxy-4H-benzo[1,4]oxazin-3-on
1-(4-Ethoxy-carbonylamino-3-cyano-5-fluorophenyl)-2-(tert-butylamino)ethanol
2-Hydroxy-5-(1-hydroxy-2-{2-[4-(2-hydroxy-2-phenyl-ethylamino)-phenyl]-ethylamino}-ethyl)-benzaldehyd
N-[2-Hydroxy-5-(1-hydroxy-2-{2-[4-(2-hydroxy-2-phenyl-ethylamino)-phenyl]-ethylamino}-ethyl)-phenyl]-formamid
8-Hydroxy-5-(1-hydroxy-2-{2-[4-(6-methoxy-biphenyl-3-ylamino)-phenyl]-ethylaminol-ethyl}-1H-quinolin-2-on
8-Hydroxy-5-[1-hydroxy-2-(6-phenethylamino-hexylamino)-ethyl]-1H-quinolin-2-one
5-[2-(2-{4-[4-(2-Amino-2-methyl-propoxy)-phenylamino]-phenyl}-ethylamino)-1-hydroxy-ethyl]-8-hydroxy-1H-quinolin-2-on
[3-(4-{6-[2-Hydroxy-2-(4-hydroxy-3-hydroxymethyl-phenyl)-ethylamino]-hexyloxy}-butyl)-5-methyl-phenyl]-hamstoff
4-(2-{6-[2-(2,6-Dichloro-benzyloxy)-ethoxy]-hexylaminol-1-hydroxy-ethyl)-2-hydroxymethyl-phenol
3-(4-{6-[2-Hydroxy-2-(4-hydroxy-3-hydroxymethyl-phenyl)-ethylamino]-hexyloxy}-butyl)-benzenesulfonamid
3-(3-{7-[2-Hydroxy-2-(4-hydroxy-3-hydroxymethyl-phenyl)-ethylamino]-heptyloxy}-propyl)-benzenesulfonamid
4-(2-{6-[4-(3-Cyclopentanesulfonyl-phenyl)-butoxy]-hexylamino}-1-hydroxy-ethyl)-2-hydroxymethyl-phenol
N-Adamantan-2-yl-2-(3-{2-[2-hydroxy-2-(4-hydroxy-3-hydroxymethyl-phenyl)-ethylamino]-propyl}-phenyl)-acetamid
(R,S)-4-(2-{[6-(2,2-Difluoro-4-phenylbutoxy)hexyl]amino}-1-hydroxy-ethyl)-2-(hydroxymethyl)phenol
(R,S)-4-(2-{[6-(2,2-Difluoro-2-phenylethoxy)hexyl]amino}-1-hydroxy-ethyl)-2-(hydroxymethyl)phenol
(R,S)-4-(2-{[4,4-Difluoro-6-(4-phenylbutoxy)hexyl]amino}-1-hydroxy-ethyl)-2-(hydroxymethyl)phenol
(R,S)-4-(2-{[6-(4,4-Difluoro-4-phenylbutoxy)hexyl]amino}-1-hydroxy-ethyl)-2-(hydroxymethyl)phenol
(R,S)-5-(2-([6-(2,2-Difluoro-2-phenylethoxy)hexyl]amino}-1-hydroxy-ethyl)-8-hydroxyquinolin-2(1H)-on
(R,S)-[2-({6-[2,2-Difluoro-2-(3-methylphenyl)ethoxy]hexyl}amino)-1-hydroxyethyl]-2-(hydroxymethyl)phenol
4-(1R)-2-{[6-(2,2-Difluoro-2-phenylethoxy)hexyl]amino}-1-hydroxyethyl)-2-(hydroxymethyl)phenol
(R,S)-2-(Hydroxymethyl)-4-(1-hydroxy-2-{[4,4,515-tetrafluoro-6-(3-phenylpropoxy)hexyl]amino}ethyl)phenol
(R,S)-[5-(2-{[6-(2,2-Difluoro-2-phenylethoxy)hexyl]amino}-1-hydroxy-ethyl)-2-hydroxyphenyl]formamid
(R,S)-4-[2-({6-[2-(3-Bromophenyl)-2,2-difluoroethoxy]hexyl}amino)-1-hydroxyethyl]- 2-(hydroxymethyl)phenol
(R, S)-N-[3-(1,1-Difluoro-2-{[6-({2-hydroxy-2-[4-hydroxy-3-(hydroxymethyl)phenyl]-ethyl}amino)hexyl]oxy}ethyl)phenyl]harnstoff
3-[3-(1,1-difluoro-2-{[6-({2-hydroxy-2-[4-hydroxy-3-(hydroxymethyl)phenyl]ethyl}-amino)hexyl]oxy}ethyl)phenyl]imidazolidine-2,4-dion
(R,S)-4-[2-({6-[2,2-difluoro-2-(3-methoxyphenyl)ethoxy]hexyl}amino)-1- hydroxyethyl]-2-(hydroxymethyl)phenol
5-((1R)-2-{[6-(2,2-difluoro-2-phenylethoxy)hexyl]amino}-1-hydroxyethyl)-8-hydroxyquinolin-2(1H)-on
4-((1R)-2-{[4,4-Difluoro-6-(4-phenylbutoxy)hexyl]amino}-1-hydroxy-ethyl)-2-(hydroxymethyl)phenol
(R,S)-4-(2-{[6-(3,3-Difluoro-3-phenylpropoxy)hexyl]amino}-1-hydroxy ethyl)-2-(hydroxymethyl)phenol
(R,S)-(2-{[6-(2,2-Difluoro-2-phenylethoxy)-4,4-difluorohexyl]amino}-1-hydroxyethyl)-2-(hydroxymethyl)phenol
(R,S)-4-(2-{[6-(2,2-difluoro-3-phenylpropoxy)hexyl]amino}-1-hydroxy-ethyl)-2-(hydroxymethyl)phenol
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus: GSK233705B, GSK573719, AD-237, ALKS-27, LAS-34273, LAS-35201, CHF-5407, QAT-370 und Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 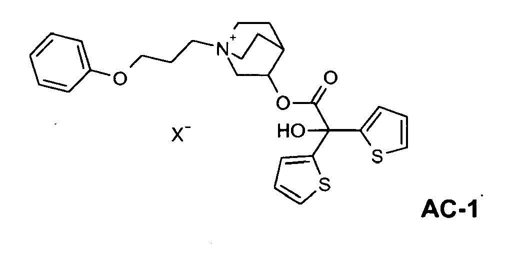 worin X- ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 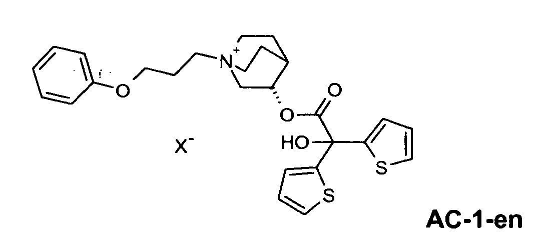 enthalten, worin X- die vorstehend genannten Bedeutungen aufweisen kann. Weiterhin bevorzugte Anticholinergika sind ausgewählt aus den Salzen der Formel **AC-2** 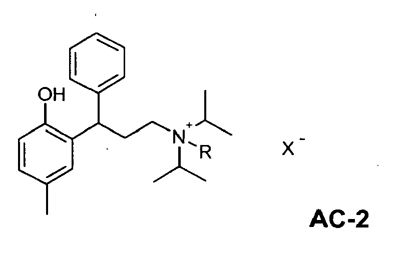 worin R entweder Methyl oder Ethyl bedeuten und worin X- die vorstehend genannte Bedeutungen aufweisen kann. In einer alternativen Ausführungsform kann die Verbindung der Formel AC-2 auch in Form der freien Base **AC-2-base** vorliegen.

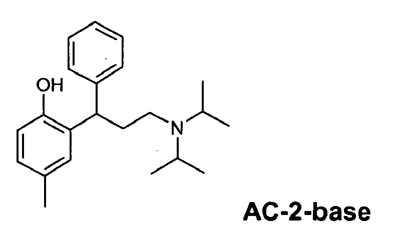

Weiterhin genannte Verbindungen sind:
2,2-Diphenylpropionsäuretropenolester-Methobromid
2,2-Diphenylpropionsäurescopinester-Methobromid
2-Fluor-2,2-Diphenylessigsäurescopinester-Methobromid
2-Fluor-2,2-Diphenylessigsäuretropenolester-Methobromid
3,3',4,4'-Tetrafluorbenzilsäuretropenolester-Methobromid
3,3',4,4'-Tetrafluorbenzilsäurescopinester-Methobromid
4,4'-Difluorbenzilsäuretropenolester-Methobromid
4,4'-Difluorbenzilsäurescopinester-Methobrom id
3,3'-Difluorbenzilsäuretropenolester-Methobromid
3,3'-Difluorbenzilsäurescopinester-Methobromid
9-Hydroxy-fluoren-9-carbonsäuretropenolester-Methobromid
9-Fluor-fluoren-9-carbonsäuretropenolester-Methobromid
9-Hydroxy-fluoren-9-carbonsäurescopinester-Methobromid
9-Fluor-fluoren-9-carbonsäurescopinester-Methobromid
9-Methyl-fluoren-9-carbonsäuretropenolester-Methobromid
9-Methyl-fluoren-9-carbonsäurescopinester-Methobromid
Benzilsäurecyclopropyltropinester-Methobromid
2,2-Diphenylpropionsäurecyclopropyltropinester-Methobromid
9-Hydroxy-xanthen-9-carbonsäurecyclopropyltropinester-Methobromid
9-Methyl-fluoren-9-carbonsäurecyclopropyltropinester-Methobromid
9-Methyl-xanthen-9-carbonsäurecyclopropyltropinester-Methobromid
9-Hydroxy-fluoren-9-carbonsäurecyclopropyltropinester-Methobromid
4,4'-Difluorbenzilsäuremethylestercyclopropyltropinester-Methobromid
9-Hydroxy-xanthen-9-carbonsäuretropenolester-Methobromid
9-Hydroxy-xanthen-9-carbonsäurescopinester-Methobromid
9-Methyl-xanthen-9-carbonsäuretropenolester-Methobromid
9-Methyl-xanthen-9-carbonsäurescopinester-Methobromid
9-Ethyl-xanthen-9-carbonsäuretropenolester-Methobromid
9-Difluormethyl-xanthen-9-carbonsäuretropenolester-Methobromid
9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X- genannten Bedeutungen haben kann.

Als Corticosteroide gelangen hierbei vorzugsweise Verbindungen zur Anwendung, die ausgewählt sind aus der Gruppe bestehend aus: Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, CP-4112, NCX-1020, NCX-1024, NS-126, PLD-177, PL-2146 QAE-397 und
6,9-Difluor-17-[(2-furanylcarbonyl)oxy]-11-hydroxy-16-methyl-3-oxo-androsta-1,4-dien-17-carbothionsäure (S)-fluoromethylester
6,9-Difluor-11-hydroxy-16-methyl-3-oxo-17-propionyloxy-androsta-1,4-dien-17-carbothionsäure (S)-(2-oxo-tetrahydro-furan-3S-yl)ester,
6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Apremilast, Arofyllin, Atizoram, Oglemilastum, Tetomilast, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370, GSK256066, ELB-353, ELB-526, GRC-4039, HT-0712, L-826141 und
N-(3,5-Dichloro-1-oxo-pyridin-4-yl)-4-difluormethoxy-3-cyclopropylmethoxybenzamid (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
(R)-(+)-1-(4-Brombenzyl)-4-[(3-cyclopentyloxy)-4-methoxyphenyl]-2-pyrrolidon 3-(Cyclopentyloxy-4-methoxyphenyl)-1-(4-N'-[N-2-cyano-S-methyl-isothioureido]benzyl)-2-pyrrolidon
cis[4-Cyano-4-(3-cyclopentyloxy-4-methoxyphenyl)cyclohexan-1-carbonsäure]
2-carbomethoxy-4-cyano-4-(3-cyclopropylmethoxy-4-difluoromethoxyphenyl)cyclohexan-1-on
cis[4-Cyano-4-(3-cyclopropylmethoxy-4-difluormethoxyphenyl)cyclohexan-1-ol]
(R)-(+)-Ethyl[4-(3-cyclopentyloxy-4-methoxyphenyl)pyrrolidin-2-yliden]acetat (S)-(-)-Ethyl[4-(3-cyclopentyloxy-4-methoxyphenyl)pyrrolidin-2-yliden]acetat
9-Cyclopentyl-5,6-dihydro-7-ethyl-3-(2-thienyl)-9*H*-pyrazolo[3,4-c]-1,2,4-triazolo[4,3-a]pyridin
9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
4-[(3-Chlor-4-fluorphenyl)amino]-6-([4-(morpholin-4-yl)-1-oxo-2-buten-1-yl]amino}-7-cyclopropylmethoxy-chinazol in
4-[(3-Chlor-4-fluorphenyl)amino]-6-([4-(N,N-diethylamino)-1-oxo-2-buten-1-yl]amino)-7-cyclopropylmethoxy-chinazolin
4-[(3-Chlor-4-fluorphenyl)amino]-6-{[4-(N,N-dimethylamino)-1-oxo-2-buten-1-yl]amino}-7-cyclopropylmethoxy-chinazolin
4-[(R)-(1-Phenyl-ethyl)amino]-6-{[4-(morpholin-4-yl)-1-oxo-2-buten-1-yl]amino}-7-cyclopentyloxy-chinazolin
4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
4-[(3-Chlor-4-fluor-phenyl)amino]-6-[2-((S)-6-methyl-2-oxo-morpholin-4-yl)-ethoxy]-7-methoxy-chinazol in
4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
4-[(3-Chlor-4-fluorphenyl)amino]-6-{[4-(N,N-dimethylamino)-1-oxo-2-buten-1-yl]amino}-7-cyclopentyloxy-chinazolin
4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
4-[(3-Ethinyl-phenyl)amino]-6,7-bis-(2-methoxy-ethoxy)-chinazolin
4-[(3-Chlor-4-fluorphenyl)amino]-7-[3-(morpholin-4-yl)-propyloxy]-6-[(vinylcarbonyl)amino]-chinazolin
4-[(R)-(1-Phenyl-ethyl)amino]-6-(4-hydroxy-phenyl)-7H-pyrrolo[2,3-d]pyrimidin 3-Cyano-4-[(3-chlor-4-fluorphenyl)amino]-6-{[4-(N,N-dimethylamino)-1-oxo-2-buten-1-yl]amino}-7-ethoxy-chinolin
4-{[3-Chlor-4-(3-fluor-benzyloxy)-phenyl]amino}-6-(5-{[(2-methansulfonyl-ethyl)amino]methyl}-furan-2-yl)chinazolin
4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
4-[(3-Ethinyl-phenyl)amino]-6-{[4-(5,5-dimethyl-2-oxo-morpholin-4-yl)-1-oxo-2-buten-1-yl]amino}-chinazolin
4-[(3-Chlor-4-fluor-phenyl)amino]-6-[2-(2,2-dimethyl-6-oxo-morpholin-4-yl)-ethoxy]-7-methoxy-chinazolin
4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
4-[(3-Chlor-4-fluor-phenyl)amino]-6-{2-[4-(2-oxo-morpholin-4-yl)-piperidin-1-yl]-ethoxy}-7-methoxy-chinazolin
4-[(3-Chlor-4-fluor-phenyl)amino]-6-[1-(tert.-butyloxycarbonyl)-piperidin-4-yloxy]-7-methoxy-chinazolin
4-[(3-Chlor-4-fluor-phenyl)amino]-6-(trans-4-amino-cyclohexan-1-yloxy)-7-methoxy-chinazolin
4-[(3-Chlor-4-fluor-phenyl)amino]-6-(trans-4-methansulfonylamino-cyclohexan-1-yloxy)-7-methoxy-chinazolin
4-[(3-Chlor-4-fluor-phenyl)amino]-6-(tetrahydropyran-3-yloxy)-7-methoxy-chinazolin
4-[(3-Chlor-4-fluor-phenyl)amino]-6-(1-methyl-piperidin-4-yloxy)-7-methoxy-chinazolin
4-[(3-Chlor-4-fluor-phenyl)amino]-6-{1-[(morpholin-4-yl)carbonyl]-piperidin-4-yloxy}-7-methoxy-chinazolin
4-[(3-Chlor-4-fluor-phenyl)amino]-6-{1-[(methoxymethyl)carbonyl]-piperidin-4-yloxy}-7-methoxy-chinazolin
4-[(3-Chlor-4-fluor-phenyl)amino]-6-(piperidin-3-yloxy)-7-methoxy-chinazolin
4-[(3-Chlor-4-fluor-phenyl)amino]-6-[1-(2-acetylamino-ethyl)-piperidin-4-yloxy]-7-methoxy-chinazolin
4-[(3-Chlor-4-fluor-phenyl)amino]-6-(tetrahydropyran-4-yloxy)-7-ethoxy-chinazolin
4-[(3-Chlor-4-fluor-phenyl)amino]-6-((S)-tetrahydrofuran-3-yloxy)-7-hydroxy-chinazolin
4-[(3-Chlor-4-fluor-phenyl)amino]-6-(tetrahydropyran-4-yloxy)-7-(2-methoxy-ethoxy)-chinazolin
4-[(3-Chlor-4-fluor-phenyl)amino]-6-{trans-4-[(dimethylamino)sulfonylamino]-cyclohexan-1-yloxy}-7-methoxy-chinazolin
4-[(3-Chlor-4-fluor-phenyl)amino,]-6-{trans-4-[(morpholin-4-yl)carbonylamino]-cyclohexan-1-yloxyl-7-methoxy-chinazolin
4-[(3-Chlor-4-fluor-phenyl)amino]-6-{trans-4-[(morpholin-4-yl)sulfonylamino]-cyclohexan-1-yloxy}-7-methoxy-chinazolin
4-[(3-Chlor-4-fluor-phenyl)amino]-6-(tetrahydropyran-4-yloxy)-7-(2-acetylamino-ethoxy)-chinazolin
4-[(3-Chlor-4-fluor-phenyl)amino]-6-(tetrahydropyran-4-yloxy)-7-(2-methansulfonylamino-ethoxy)-chinazolin
4-[(3-Chlor-4-fluor-phenyl)amino]-6-{1-[(piperidin-1-yl)carbonyl]-piperidin-4-yloxy}-7-methoxy-chinazolin
4-[(3-Chlor-4-fluor-phenyl)amino]-6-(1-aminocarbonylmethyl-piperidin-4-yloxy)-7-methoxy-chinazolin
4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
4-[(3-Chlor-4-fluor-phenyl)amino]-6-(trans-4-ethansulfonylamino-cyclohexan-1-yloxy)-7-methoxy-chinazolin
4-[(3-Chlor-4-fluor-phenyl)amino]-6-(1-methansulfonyl-piperidin-4-yloxy)-7-ethoxy-chinazolin
4-[(3-Chlor-4-fluor-phenyl)amino]-6-(1-methansulfonyl-piperidin-4-yloxy)-7-(2-methoxy-ethoxy)-chinazolin
4-[(3-Chlor-4-fluor-phenyl)amino]-6-[1-(2-methoxy-acetyl)-piperidin-4-yloxy]-7-(2-methoxy-ethoxy)-chinazolin
4-[(3-Chlor-4-fluor-phenyl)amino]-6-(cis-4-acetylamino-cyclohexan-1-yloxy)-7-methoxy-chinazolin
4-[(3-Ethinyl-phenyl)amino]-6-[1-(tert.-butyloxycarbonyl)-piperidin-4-yloxy]-7-methoxy-ch inazol in
4-[(3-Ethinyl-phenyl)amino]-6-(tetrahydropyran-4-yloxy]-7-methoxy-chinazolin
4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
4-[(3-Chlor-4-fluor-phenyl)amino]-6-{cis-4-[(morpholin-4-yl)carbonylamino]-cyclohexan-1-yloxy}-7-methoxy-chinazolin
4-[(3-Chlor-4-fluor-phenyl)amino]-6-{1-[2-(2-oxopyrrolidin-1-yl)ethyl]-piperidin-4-yloxy}-7-methoxy-chinazolin
4-[(3-Chlor-4-fluor-phenyl)amino]-6-{1-[(morpholin-4-yl)carbonyl]-piperidin-4-yloxy}-7-(2-methoxy-ethoxy)-chinazol in
4-[(3-Ethinyl-phenyl)amino]-6-(1-acetyl-piperidin-4-yloxy)-7-methoxy-chinazolin
4-[(3-Ethinyl-phenyl)amino]-6-(1-methyl-piperidin-4-yloxy)-7-methoxy-chinazol in
4-[(3-Ethinyl-phenyl)amino]-6-(1-methansulfonyl-piperidin-4-yloxy)-7-methoxy-chinazolin
4-[(3-Chlor-4-fluor-phenyl)amino]-6-(1-methyl-piperidin-4-yloxy)-7(2-methoxy-ethoxy)-chinazolin
4-[(3-Chlor-4-fluor-phenyl)amino]-6-(1-isopropyloxycarbonyl-piperidin-4-yloxy)-7-methoxy-chinazolin
4-[(3-Chlor-4-fluor-phenyl)amino]-6-(cis-4-methylamino-cyclohexan-1-yloxy)-7-methoxy-chinazolin
4-[(3-Chlor-4-fluor-phenyl)amino]-6-{cis-4-[N-(2-methoxy-acetyl)-N-methyl-amino]-cyclohexan-1-yloxy}-7-methoxy-chinazolin
4-[(3-Ethinyl-phenyl)amino]-6-(piperidin-4-yloxy)-7-methoxy-chinazolin 4-[(3-Ethinyl-phenyt)amino]-6-[1-(2-methoxy-acetyl)-piperidin-4-yloxy]-7-methoxy-chinazolin
4-[(3-Ethinyl-phenyl)amino]-6-{1-[(morpholin-4-yl)carbonyl]-piperidin-4-yloxy}-7-methoxy-chinazolin
4-[(3-Chlor-4-fluor-phenyl)amino]-6-{1-[(cis-2,6-dimethyl-morpholin-4-yl)carbonyl]-piperidin-4-yloxy}-7-methoxy-chinazolin
4-[(3-Chlor-4-fluor-phenyl)amino]-6-{1-[(2-methyl-morpholin-4-yl)carbonyl]-piperidin-4-yloxy}-7-methoxy-chinazolin
4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
4-[(3-Chlor-4-fluor-phenyl)amino]-6-{1-[(N-methyl-N-2-methoxyethyl-amino)carbonyl]-piperidin-4-yloxy}-7-methoxy-chinazolin
4-[(3-Chlor-4-fluor-phenyl)amino]-6-(1-ethyl-piperidin-4-yloxy)-7-methoxy-chinazolin 4-[(3-Chlor-4-fluor-phenyl)amino]-6-{1-[(2-methoxyethyl)carbonyl]-piperidin-4-yloxy}-7-methoxy-chinazolin
4-[(3-Chlor-4-fluor-phenyl)amino]-6-{1-[(3-methoxypropyl-amino)-carbonyl]-piperidin-4-yloxy}-7-methoxy-chinazolin
4-[(3-Chlor-4-fluor-phenyl)amino]-6-[cis-4-(N-methansulfonyl-N-methyl-amino)-cyclohexan-1-yloxy]-7-methoxy-chinazolin
4-[(3-Chlor-4-fluor-phenyl)amino]-6-[cis-4-(N-acetyl-N-methyl-amino)-cyclohexan-1-yloxy]-7-methoxy-chinazolin
4-[(3-Chlor-4-fluor-phenyl)amino]-6-(trans-4-methylamino-cyclohexan-1-yloxy)-7-methoxy-chinazolin
4-[(3-Chlor-4-fluor-phenyl)amino]-6-[trans-4-(N-methansulfonyl-N-methyl-amino)-cyclohexan-1-yloxy]-7-methoxy-chinazolin
4-[(3-Chlor-4-fluor-phenyl)amino]-6-(trans-4-dimethylamino-cyclohexan-1-yloxy)-7-methoxy-chinazolin
4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazol in
4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
4-[(3-Chlor-4-fluor-phenyl)amino]-6-(1-methansulfonyl-piperidin-4-yloxy)-7-methoxy-chinazolin
4-[(3-Chlor-4-fluor-phenyl)amino]-6-(1-cyano-piperidin-4-yloxy)-7-methoxy-chinazolin gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Rezeptor Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als PAF-Antagonisten gelangen hierbei vorzugsweise Verbindungen zur Anwendung, die ausgewählt sind aus der Gruppe bestehend aus lexipafant und 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin
6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß, bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTB4-Rezeptor Antagonisten gelangen hierbei vorzugsweise Verbindungen zur Anwendung, die ausgewählt sind aus der Gruppe bestehend aus AM-103, BIIL 284 und BIIL260 gegebenenfalls in Form ihrer Racemate, Enantiomere, Diastereomere und gegebenenfalls in Form ihrer pharmakologisch verträglichen Säureadditionssalze, Solvate, Prodrugs oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Rezeptor Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, CR-3465, ONO-RS-531, L-733321, BAY-u9773 und 1-(((R)-(3-(2-(6,7-Difluor-2-quinolinyl)ethenyl)phenyl)-3-(2-(2-hydroxy-2-propyl)phenyl)thio)methylcyclopropan-essigsäure, 1-(((1 (R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in-Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als Histamin H1 Rezeptor Antagonisten gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Histamin H4 Rezeptor Antagonisten gelangen hierbei vorzugsweise Verbindungen zur Anwendung, die ausgewählt sind aus der Gruppe bestehend aus: JNJ-7777120 gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als Inhibitoren von Nicht-Rezeptor Tyrosine Kinasen wie zum Beispiel LYN, LCK, SYK, ZAP-70, FYN, BTK oder ITK gelangen hierbei vorzugsweise Verbindungen zur Anwendung, die ausgewählt sind aus der Gruppe bestehend aus
2-[(2-aminoethyl)amino]-4-[(3-bromophenyl)amino]-5-Pyrimidinecarboxamid;
2-[[7-(3,4-dimethoxyphenyl)imidazo[1,2-c]pyrimidin-5-yl]amino]-3-Pyridinecarboxamid;
6-[[5-fluoro-2-[3,4,5-trimethoxyphenyl)amino]-4-pyrimidinyl]amino]-2,2-dimethyl-2H-Pyrido[3,2-b]-1,4-oxazin-3(4H)-on;
N-[3-bromo-7-(4-methoxyphenyl)-1,6-naphthyridin-5-yl]-1,3-Propanediamin
7-(4-methoxyphenyl)-N-methyl-1,6-Naphthyridin-5-amin;
N-[7-(4-methoxyphenyl)-1,6-naphthyridin-5-yl]-1,3-Propanediamin;
N-[7-(2-thienyl)-1,6-naphthyridin-5-yl-1,3-Propanediamin;
N-[7-[4-(dimethylamino)phenyl]-1,6-naphthyridin-5-yl]-1,2-Ethanediamin;
N-[7-(4-methoxyphenyl)-2-(trifluoromethyl)-1,6-naphthyridin-5-yl]- 1,3-Propanediamin;
N-[7-(4-methoxyphenyl)-3-phenyl-1,6-naphthyridin-5-yl]-1,3-Propanediamin;
N-(7-phenyl-1,6-naphthyridin-5-yl)-1,3-Propanediamin;
N-[7-(3-fluorophenyl)-1,6-naphthyridin-5-yl]-1,3-Propanediamin;
N-[7-(3-chlorophenyl)-1,6-naphthyridin-5-yl]-1,3-Propanediamin;
N-[7-[3-(trifluoromethoxy)phenyl]-1,6-naphthyridin-5yl]-1,3-Propanediamin;
N-[7-(4-fluorophenyl)-1,6-naphthyridin-5-yl]-1,3-Propanediamin;
N-[7-(4-fluorophenyl)-1,6-naphthyridin-5-yl]-1,3-Propanediamin;
N-[7-(4-chlorophenyl)-1,6-naphthyridin-5-yl]-1,3-Propanediamin;
N-[7-(4'-methyl[1,1'-biphenyl]-4-yl)-1,6-naphthyridin-1,3-Propanediamin;
N-[7-[4-(dimethylamino)phenyl]-1,6-naphthyridin-5-yl]-1,3-Propanediamin;
N-[7-[4-(diethylamino)phenyl]-1,6-naphthyridin-5-yl]-1,3-Propanediamin;
N-[7-[4-(4-morpholinyl)phenyl]-1,6-naphthyridin-5-yl]-1,3-Propanediamin;
N-[7-[4-[[2-(dimethylamino)ethyl]methylamino]phenyl]-1,6-naphthyridin-5-yl]-1,3-Propanediamin;
N-[7-(4-bromophenyl)-1,6-naphthyridin-5-yl]-1,3-Propanediamin;
N-[7-(4-methylphenyl)-1,6-naphthyridin-5-yl]-1,3-Propanediamin;
N-[7-[4-(methylthio)phenyl]-1,6-naphthyridin-5-yl]-1,3-Propanediamin;
N-[7-[4-(1-methylethyl)phenyl]-1,6-naphthyridin-5-yl]-1,3-Propanediamin;
7-[4-(dimethylamino)phenyl]-N-methyl-1,6-Naphthyridin-5-amin;
7-[4-(dimethylamino)phenyl]-N,N-dimethyl-1,6-Naphthyridin-5-amin;
N-[7-[4-(dimethylamino)phenyl]-1,6-naphthyridin-5-yl]-1,4-Butanediamin;
N-[7-[4-(dimethylamino)phenyl]-1,6-naphthyridin-5-yl]-1,5-Pentanediamin;
3-[[7-[4-(dimethylamino)phenyl]-1,6-naphthyridin-5-yl]oxy]-1-Propanol;
4-[5-(4-aminobutoxy)-1,6-naphthyridin-7-yl]-N,N-dimethyl-Benzenamin;
4-[[7-[4-(dimethylamino)phenyl]-1,6-naphthyridin-5-yl]amino]-1-Butanol;
N-[7-[4-(dimethylamino)phenyl]-1,6-naphthyridin-5-yl]-N-methyl-1,3-Propanediamin;
N-[7-[4-(dimethylamino)phenyl]-1,6-naphthyridin-5-yl]-N'-methyl-1,3-Propanediamin;
N-[7-[4-(dimethylamino)phenyl]-1,6-naphthyridin-5-yl]-N,N'-dimethyl-1,3-Propanediamin;
1-amino-3-[[7-[4-(dimethylamino)phenyl]-1,6-naphthyridin-5-yl]amino]-2-Propanol;
N-[7-[4-(dimethylamino)phenyl]-1,6-naphthyridin-5-yl]-2,2-dimethyl-1,3-Propanediamin;
7-[4-(dimethylamino)phenyl]-N-(3-pyridinylmethyl)-1,6-Naphthyridin-5-amin;
N-[(2-aminophenyl)methyl]-7-[4-(dimethylamino)phenyl]-1,6-Naphthyridin-5-amin;
N-[7-[6-(dimethylamino)[1,1'-biphenyl]-3-yl]-1,6-naphthyridin-5-yl]-1,3-Propanediamin;
N-[7-[3-chloro-4-(diethylamino)phenyl]-1,6-naphthyridin-5-yl]-1,3-Propanediamin ;
N-[7-[4-(dimethylamino)-3-methoxyphenyl]-1,6-naphthyridin-5-yl]-1,3-Propänediamin;
N-[7-[4-(diethylamino)phenyl]-3-methyl-1,6-naphthyridin-5-yl]-1,3-Propanediamin;
N-[7-(3'-fluoro[1,1'-biphenyl]-3-yl)-1,6-naphthyridin-5-yl]-1,2-Ethanediamin,
N-[7-(4-methoxyphenyl)-1,6-naphthyridin-5-yl]-1,6-Naphthyridine-1,3-Propanediamin;
N,N'-bis(3-aminopropyl)-7-(4-methoxyphenyl)-2,5-diamin;
N-[7-(4-methoxyphenyl)-2-(phenylmethoxy)-1,6-naphthyridin-5-yl]-1,6-Naphthyridine-1,3-Propanediamin;
N5-(3-aminopropyl)-7-(4-methoxyphenyl)-N2-(phenylmethyl)-2,5-diamin;
N-[7-(2-naphthalenyl)-1,6-naphthyridin-5-yl]-1,3-Propanediamin;
N-[7-(2'-fluoro[1,1'-biphenyl]-4-yl)-1,6-naphthyridin-5-yl]-1,3-Propanediamin;
N-[7-(3,4,5-trimethoxyphenyl)-1,6-naphthyridin-5-yl]-1,3-Propanediamin;
N-[7-(3,4-dimethylphenyl)-1,6-naphthyridin-5-yl]-1,3-Propanediamin;
1-amino-3-[[7-(2-naphthalenyl)-1,6-naphthyridin-5-yl]amino]-2-Propanol;
1-amino-3-[[7-(2'-fluoro[1,1'-biphenyl]-4-yl)-1,6-naphthyridin-5-yl]amino]-2-Propanol;
1-amino-3-[[7-(4'-methoxy[1,1'-biphenyl]-4-yl)-1,6-naphthyridin-5-yl]amino]-2-Propanol;
1-amino-3-[[7-(3,4,5-trimethoxyphenyl)-1,6-naphthyridin-5-yl]amino]-2-Propanol;
1-amino-3-[[7-(4-bromophenyl)-1,6-naphthyridin-5-yl]amino]-2-Propanol;
N-[7-(4'-methoxy[1,1'-biphenyl]-4-yl)-1,6-naphthyridin-5-yl]-2,2-dimethyl-1,3-Propanediamin;
1-[[7-[4-(dimethylamino)phenyl]-1,6-naphthyridin-5-yl]amino]-2-Propanol;
2-[[2-[[7-[4-(dimethylamino)phenyl]-1,6-naphthyridin-5-yl]amino]ethyl]thio]-Ethanol;
7-[4-(dimethylamino)phenyl]-N-(3-methyl-5-isoxazolyl)-1,6-Naphthyridin-5-amin;
7-[4-(dimethylamino)phenyl]-N-4-pyrimidinyl-1,6-Naphthyridin-5-amin;
N-[7-[4-(dimethylamino)phenyl]-1,6-naphthyridin-5-yl]-1,3-Cyclohexanediamin;
N,N-dimethyl-4-[5-(1-piperazinyl)-1,6-naphthyridin-7-yl]-Benzenamin;
4-[5-(2-methoxyethoxy)-1,6-naphthyridin-7-yl]-N,N-dimethyl-Benzenamin;
1-[7-[4-(dimethylamino)phenyl]-1,6-naphthyridin-5-yl]-4-Piperidinol;
1-[7-[4-(dimethylamino)phenyl]-1,6-naphthyridin-5-yl]-3-Pyrrolidinol;
7-[4-(dimethylamino)phenyl]-N-(2-furanylmethyl)-1,6-Naphthyridin-5-amin;
7-[4-(dimethylamino)phenyl]-N-[3-(1H-imidazol-1-yl)propyl]-1,6-Naphthyridin-5-amin;
1-[7-[4-(dimethylamino)phenyl]-1,6-naphthyridin-5-yl]-4-Piperidinecarboxamid;
1-[3-[[7-[4-(dimethylamino)phenyl]-1,6-naphthyridin-5-yl]amino]propyl]-2-Pyrrol idinon;
N-[3'-[5-[(3-aminopropyl)amino]-1,6-naphthyridin-7-yl][1,1'-biphenyl]-3-yl]-Acetamid;
N-[7-(4'-fluoro[1,1'-biphenyl]-4-yl)-1,6-naphthyridin-5-yl]-1,3-Propanediamin;
N-[4'-[5-[(3-aminopropyl)amino]-1,6-naphthyridin-7-yl][1,1'-biphenyl]-3-yl]-Acetamid;
N-[7-[4-(1,3-benzodioxol-5-yl)phenyl]-1,6-naphthyridin-5-yl]-1,3-Propanediamin;
N-[7-[4-(2-thienyl)phenyl]-1,6-naphthyridin-5-yl]-1,3-Propanediamin;
N-[7-[4-fluoro-3-(trifluoromethyl)phenyl]-1,6-naphthyridin-5-yl]-1,3-Propanediamin;
N-[7-[4-(3-pyridinyl)phenyl]-1,6-naphthyridin-5-yl]-1,3-Propanediamin;
N-[7-(1,3-benzodioxol-5-yl)-1,6-naphthyridin-5-yl]-1,3-Propanediamin;
N-[7-(6-methoxy-2-naphthalenyl)-1,6-naphthyridin-5-yl]-1,3-Propanediamin;
7-[4-(dimethylamino)phenyl]-N-(4-pyridinylmethyl)-1,6-Naphthyridin-5-amin;
3-[[7-[4-(dimethylamino)phenyl]-1,6-naphthyridin-5-yl]methylamino]-Propanenitril;
7-[4-(dimethylamino)phenyl]-N-[1-(phenylmethyl)-4-piperidinyl]-1,6-Naphthyridin-5-amin;
N-[7-[4-(dimethylamino)phenyl]-1,6-naphthyridin-5-yl]-1,2-Cyclohexanediamin. gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als MAP Kinase Inhibitoren gelangen hierbei vorzugsweise Verbindungen zur Anwendung, die ausgewählt sind aus der Gruppe bestehend aus: SCIO-323, SX-011, SD-282, SD-169, N PC-037282, SX-004, VX-702, GSK-681323, GSK-856553, ARRY-438162, ARRY-p38-002, ARRY-371797, AS-602801, AS-601245, AS-602183, CEP-1347, KC706, TA-5493, RO-6226, Ro-1487, SC-409 und BIRB-796 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Inhibitoren des NF-κB Signalwegs oder der IKK Kinasen gelangen hierbei vorzugsweise Verbindungen zur Anwendung, die ausgewählt sind aus der Gruppe bestehend aus: MD-1041, MLN-041 und AVE-0547 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als iNOS-Inhibitoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, AMT, L-Canavanin, 2-lminopiperidin, S-Isopropylisothioharnstoff, S-Methylisothioharnstoff, S-Ethylisothioharnstoff, S-Methyltiocitrullin, S-Ethylthiocitrullin, L-NA (N^{ω}-Nitro-L-arginin), L-NAME (N^{ω}-Nitro-L-argininmethylester), L-NMMA (N^{ω}-Monomethyl-L-arginin), L-NIO-(N^{ω}-Iminoethyl-L-ornithin), L-NIL (N^{ω}-Iminoethyl-lysin), (S)-6-Acetimidoylamino-2-amino-hexanoic acid (1*H*-tetrazol-5-yl)-amid (SC-51), 1400W, (S)-4-(2-Acetimidoylamino-ethylsulfanyl)-2-amino-buttersäure (GW274150), 2-[2-(4-Methoxy-pyridin-2-yl)-ethyl]-3*H*-imidazo[4,5-*b*]pyridin (BYK191023), 2-((R)-3-Amino-1-phenyl-propoxy)-4-chlor-5-fluorbenzonitril, 2-((1 R,3S)-3-Amino-4-hydroxy-1-thiazol-5-yl-butylsulfanyl)-6-trifluoromethyl-nicotinonitril, 2-((1R,3S)-3-Amino-4-hydroxy-1-thiazol-5-yl-butylsulfanyl)-4-chlor-benzonitril, 2-((1 R,3S)-3-Amino-4-hydroxy-1-thiazol-5-yl-butylsulfanyl)-5-chlor-benzonitril, (2S,4R)-2-Amino-4-(2-chlor-5-trifluoromethyl-phenylsulfanyl)-4-thiazol-5-yl-butan-1-ol, 2-((1R,3S)-3-Amino-4-hydroxy-1-thiazol-5-yl-butylsulfanyl)-5-chlor-nicotinonitril, 4-((S)-3-Amino-4-hydroxy-1-phenyl-butylsulfanyl)-6-methoxy-nicotinonitril, substituierte 3-Phenyl-3,4-dihydro-1-isoquinolinamin wie z.B. AR-C102222, (1S,5S,6R)-7-Chlor-5-methyl-2-aza-bicyclo[4.1.0]hept-2-en-3-ylamin (ONO-1714), (4R,5R)-5-Ethyl-4-methyl-thiazolidin-2-ylideneamin, (4R,5R)-5-Ethyl-4-methyl-selenazolidin-2-ylideneamin, 4-Aminotetrahydrobiopterin, (E)-3-(4-Chlor-phenyl)-*N*-(1-{2-oxo-2-[4-(6-trifluormethyl-pyrimidin-4-yloxy)-piperidin-1-yl]-ethylcarbamoyl}-2-pyridin-2-yl-ethyl)-acrylamid (FR260330), 3-(2,4-Difluor-phenyl)-6-[2-(4-imidazol-1-ylmethyl-phenoxy)-ethoxy]-2-phenyl-pyridin (PPA250), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) und deren pharmazeutischen Salze, Prodrugs oder Solvate. Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-*S*-glutathione, Estradiol 17-β-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glycocholate, Glycolithocholic acid sulphate, Ibuprofen, Indomethacin, Indoprofen, Ketoprofen, Lithocholic acid sulphate, Methotrexate, MK571 ((*E*)-3-[[[3-[2-(7-Chloro-2-quinolinyl)ethenyl]phenyl]-[[3-dimethylamino)-3-oxopropyl]thio]methyl]thio]-propanoic acid), α-Naphthyl-β-D-glucuronide, Nitrobenzyl mercaptopurine riboside, Probenecid, PSC833, Sildenafil, Sulfinpyrazone, Taurochenodeoxycholate, Taurocholate, Taurodeoxycholate, Taurolithocholate, Taurolithocholic acid sulphate, Topotecan, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.
Besonders bevorzugt sind *N*-Acetyl-dinitrophenyl-Cysteine, Dehydroepiandrosterone 3-sulphate, Dilazep, Dinitrophenyl-*S*-glutathione, Estradiol 3,17-disulphate, Flurbiprofen, Glycocholate, Glycolithocholic acid sulphate, Ibuprofen, Indomethacin, Indoprofen, Lithocholic acid sulphate, MK571, PSC833, Sildenafil, Taurochenodeoxycholate, Taurocholate, Taurolithocholate, Taurolithocholic acid sulphate, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Als Leukotriene Biosynthese Inhibitoren wie zum Beispiel aus der Gruppe der 5-Lipoxygenase (5-LO) Inhibitoren, cPLA2 Inhibitoren, Leukotriene A4 hydrolase Inhibitoren oder FLAP Inhibitoren, gelangen hierbei vorzugsweise Verbindungen zur Anwendung, die ausgewählt sind aus der Gruppe bestehend aus zileuton, tipelukast, licofelone, darapladib, TA-270, IDEA-033, IDEA-070, NIK-639, ABT-761, fenleuton, tepoxalin, Abbott-79175, Abbott-85761, PLT-3514, CMI-903, PEP-03, CMI-977, MLN-977, CMI-947, LDP-977, efipladib, PLA-695, veliflapon, MK-591, MK-886 und BAYx1005 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Nicht-steroidale antientzündliche Agenzien (NSAIDs) gelangen hierbei vorzugsweise Verbindungen zur Anwendung, die ausgewählt sind aus der Gruppe bestehend aus Piroxicam, Diclofenac, Naproxen, Flurbiprofen, Fenoprofen, Ketoprofen And Ibuprofen, Nimesulide, Indomethacin, Sulindac, Azapropazone, Phenylbutazone, Aspirin; Meloxicam, Celecoxib, Rofecoxib, Valdecoxib, Lumarocoxib, Parecoxib, Tenoxicam und Etoricoxib, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als CRTH2 Antagonisten gelangen hierbei vorzugsweise Verbindungen zur Anwendung, die ausgewählt sind aus der Gruppe bestehend aus Ramatroban, AP-761, ODC-9101, SAR-398171, SAR-389644, Laropiprant, TM-30642, TM-30643 und TM-30089 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als DP1-Rezeptor Modulatoren gelangen hierbei vorzugsweise Verbindungen zur Anwendung, die ausgewählt sind aus der Gruppe bestehend aus S-5751, laropiprant, SAR-389644 und TS-002 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Thromboxane Rezeptor Antagonist gelangen hierbei vorzugsweise Verbindungen zur Anwendung, die ausgewählt sind aus der Gruppe bestehend aus Seratrodast, BM-573, (+/-)-sodium[2-(4-chlorophenylsulfonylaminomethyl)- indan-5-yl]acetate monohydrate (Z-335) und KP-496 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Chemokine Rezeptor Antagonisten gelangen hierbei vorzugsweise Verbindungen zur Anwendung, die ausgewählt sind aus der Gruppe bestehend aus BX-471, SH-T-04268-H, MLN-3701, MLN-3897; MLX-010, MLX-025, MLX-011, MLX-031, MLX-045, AVE-0545, CP-481715, INCB-003284, INCB-8696, INCB-15050, INCB-9471, JNJ-27553292, Sch-417690, CCX-282, SB-656933, SCH-527123, SB-656933 und AMD-3100 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Neurokinin (NK1 oder NK2) Antagonisten gelangen hierbei vorzugsweise Verbindungen zur Anwendung, die ausgewählt sind aus der Gruppe bestehend aus: saredutant, Nepadutant, PRX-96026 und Figopitant gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Sphingosine1-phosphat Rezeptor Modulatoren gelangen hierbei vorzugsweise Verbindungen zur Anwendung, die ausgewählt sind aus der Gruppe bestehend aus: c-6448 und FTY720 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Mucoregulatoren gelangen hierbei vorzugsweise Verbindungen zur Anwendung, die ausgewählt sind aus der Gruppe bestehend aus: MSI-2216, Erdosteine, Fluorovent, Talniflumate, INO-4995, BIO-11006, VR-496, fudosteine und ENAC blocker 552617 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als PPAR gamma Agonist gelangen hierbei vorzugsweise Verbindungen zur Anwendung, die ausgewählt sind aus der Gruppe bestehend aus: Rosiglitazone, Ciglitazone, Pioglitazone und SMP-028 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Rho Kinase Inhibitoren gelangen hierbei vorzugsweise Verbindungen zur Anwendung, die ausgewählt sind aus der Gruppe bestehend aus: Fasudil gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Als Adenosine Rezeptor Modulatoren gelangen hierbei vorzugsweise Verbindungen zur Anwendung, die ausgewählt sind aus der Gruppe bestehend aus CGH-2466, CVT-6883, MRS-1754, UK-432097 und L-971 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate

Als Bradykinin (BK2 oder BK1) Antagonisten gelangen hierbei vorzugsweise Verbindungen zur Anwendung, die ausgewählt sind aus der Gruppe bestehend aus Icatibant und MEN-16132 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate

Als Endothelin Antagonisten gelangen hierbei vorzugsweise Verbindungen zur Anwendung, die ausgewählt sind aus der Gruppe bestehend aus Actelion-1, Ambrisentan, Sitaxsentan, TBC-3711, TBC-3214 und Bosentan gegebenenfalls in Form ihrer Racemate, Enantiomere, Diastereomere und gegebenenfalls in Form ihrer pharmakologisch verträglichen Säureadditionssalze, Prodrugs, Solvate oder Hydrate Als Interleukin 1-beta converting enzyme (ICE) Inhibitoren gelangen hierbei vorzugsweise Verbindungen zur Anwendung, die ausgewählt sind aus der Gruppe bestehend aus Pralnacasan, VRT-18858, RU-36384, VX-765 und VRT-43198 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Toll-like Rezeptor (TLR) Modulatoren gelangen hierbei vorzugsweise Verbindungen zur Anwendung, die ausgewählt sind aus der Gruppe bestehend aus Resiquimod, PF-3512676, AVE-0675, Heplisav, IMO-2055, CpG-28, TAK-242, SAR-21609, RC-52743198 und 852A gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als HMG-CoA Reductase Inhibitoren gelangen hierbei vorzugsweise Verbindungen zur Anwendung, die ausgewählt sind aus der Gruppe bestehend aus Lovastatin, Simvastatin, Pravastatin, Fluvastatin und Avorvastatin gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als VLA-4 Antagonisten gelangen hierbei vorzugsweise Verbindungen zur Anwendung, die ausgewählt sind aus der Gruppe bestehend aus Natalizumab, valategrast, TBC-4746, CDP-323 und TL-1102 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als ICAM-1 Inhibitoren gelangen hierbei vorzugsweise Verbindungen zur Anwendung, die ausgewählt sind aus der Gruppe bestehend aus BIRT-2584 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als SHIP Agonisten gelangen hierbei vorzugsweise Verbindungen zur Anwendung, die ausgewählt sind aus der Gruppe bestehend aus AQX-MN100 und MN-106 gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als TNFα Antagonisten gelangen hierbei vorzugsweise Verbindungen zur Anwendung, die ausgewählt sind aus der Gruppe bestehend aus Infliximab, Adalimumab, Golimumab. CytoFab und Etanercept gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen gegen Schwellungen der Atemwege gelangen hierbei vorzugsweise Verbindungen zur Anwendung, die ausgewählt sind aus der Gruppe bestehend aus Phenylephrine, Phenylpropanolamine, Pseudophedrine, Oxymetazoline, Epinephrine, Naphazoline, Xylometazoline, Propylhexedrine und Llevodesoxyephedrine gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen gegen Husten gelangen hierbei vorzugsweise Verbindungen zur Anwendung, die ausgewählt sind aus der Gruppe bestehend aus Hydrocodone, Caramiphen, Carbetapentane und Dextramethorphan gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0.001-100 mg/kg Körpergewicht, vorzugsweise bei 0.1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

### Herstellung der Ausgangsverbindungen:

### Beispiel I

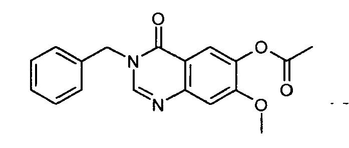

### 3-Benzyl-3,4-dihydro-4-oxo-6-acetyloxy-7-methoxy-chinazolin

169 g 3,4-Dihydro-4-oxo-6-acetyloxy-7-methoxy-chinazolin, 118.8 ml Benzylbromid und 138,2 g Kaliumcarbonat werden in 1600 ml Aceton für 8 Stunden auf 35-40°C erwärmt. Die Mischung wird 15 Stunden bei Raumtemperatur gerührt und anschließend mit 2000 ml Wasser versetzt. Die Suspension wird auf 0°C abgekühlt, der Niederschlag wird abgesaugt, mit 400 ml Wasser und 400 ml tert.-Butylmethylether gewaschen und bei 50°C getrocknet. Der Feststoff wird in 4000 ml Methylenchlorid gelöst, filtriert und eingeengt. Der Rückstand wird in tert.-Butylmethylether suspendiert, abgesaugt und bei 50°C getrocknet. Ausbeute: 203 g (86% der Theorie)
R_{f}-Wert: 0.80 (Kieselgel, Methylenchlorid/Ethanol = 9:1)
Massenspektrum (ESI⁺): m/z = 325 [M+H]⁺

### Beispiel II

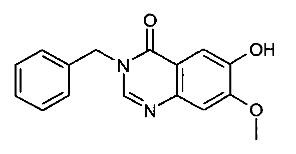

### 3-Benzyl-3,4-dihydro-4-oxo-6-hydroxy-7-methoxy-chinazolin

### Verfahren A:

168.5 g 6-Hydroxy-7-methoxy-benzo[d][1,3]oxazin-4-on werden in 1200 ml Toluol gelöst und 74.7 ml Benzylamin werden zugegeben. Die Mischung wird 15 Stunden unter Rückfluss erhitzt und danach auf Raumtemperatur abgekühlt. Der Niederschlag wird abfiltriert und mit tert.-Butylmethylether gewaschen.
Ausbeute 124 g (72% der Theorie)

### Verfahren B:

200 g 3-Benzyl-3,4-dihydro-4-oxo-6-acetyloxy-7-methoxy-chinazolin werden in 200 ml Wasser und 1000 ml Ethanol suspendiert. 300 ml 10N Natriumhydroxid Lösung werden bei Raumtemperatur zugegeben und die Mischung 1 Stunde auf 30°C erwärmt. Nach Zugabe von 172 ml Essigsäure und 2000 ml Wasser wird die Mischung 20 Stunden bei Raumtemperatur gerührt. Der Niederschlag wird abgesaugt, mit Wasser und Aceton gewaschen und bei 60°C getrocknet.
Ausbeute: 172,2 g (98% der Theorie)
R_{f}-Wert: 0.25 (Kieselgel, Methylenchlorid/Ethanol = 19:1)
Massenspektrum (ESI⁺): m/z = 283 [M+H]⁺

### Beispiel III

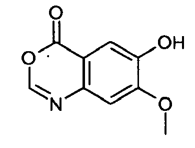

### 6-Hydroxy-7-methoxy-benzo[d][1,3]oxazin-4-on

1 g 2-Amino-5-hydroxy-4-methoxy-benzoesäure (hergestellt durch Umsetzung von 2-Nitro-4,5-dimethoxy-benzoesäure-methylester mit Kalilauge zu 2-Nitro-5-hydroxy-4-methoxy-benzoesäure-Kaliumsalz und anschließender katalytischer Hydrierung in Gegenwart von Palladium auf Aktivkohle) und 20 ml Orthoameisensäure-triethylester werden für 2.5 Stunden auf 100°C erhitzt. Nach Abkühlen auf Raumtemperatur wird der Niederschlag abgesaugt und mit Diethylether gewaschen.
Ausbeute: 0.97 g (93% der Theorie)
R_{f}-Wert: 0.86 (Kieselgel, Methylenchlorid/Methanol/Essigsäure = 90:10:1)
Massenspektrum (ESI⁺): m/z = 194 [M+H]⁺

### Beispiel IV

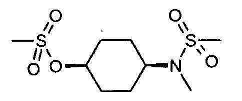

### cis-1-(Methansulfonyloxy)-4-(N-methansulfonyl-N-methyl-amino)-cyclohexan

Hergestellt durch Umsetzung von cis-1-Hydroxy-4-methylamino-cyclohexan mit Methansulfonsäurechlorid in Tetrahydrofuran in Gegenwart von Triethylamin.
Massenspektrum (ESI⁺): m/z = 286 [M+H]⁺

Analog Beispiel IV können erhalten werden:

| **Beispiel** | **Struktur** |
|---|---|
| IV(1) | 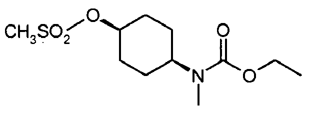 |
| IV(2) | 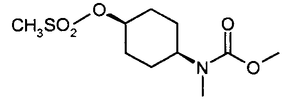 |
| IV(3) | 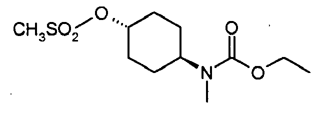 |
| IV(4) | 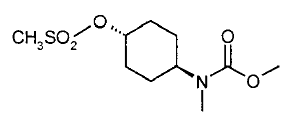 |
| IV(5) | 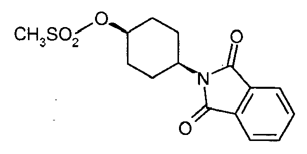 |
| IV(6) | 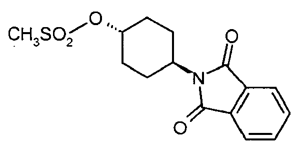 |
| IV(7) | 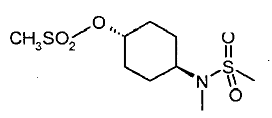 |
| IV(8) | 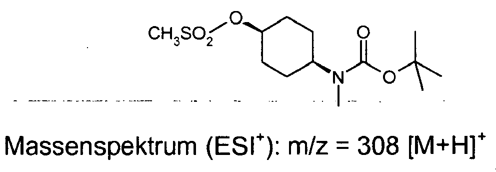 |
| IV(9) | 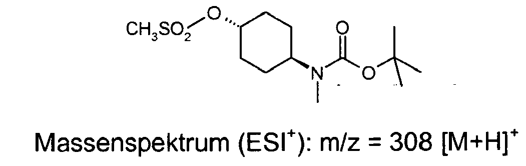 |
| IV(10) | 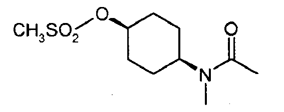 |
| IV(11) | 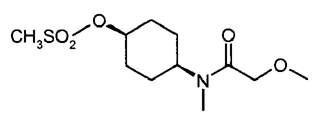 |
| IV(12) | 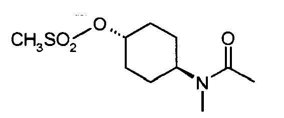 |
| IV(13) | 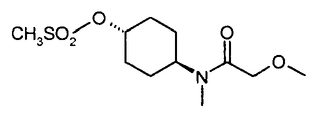 |
| IV(14) | 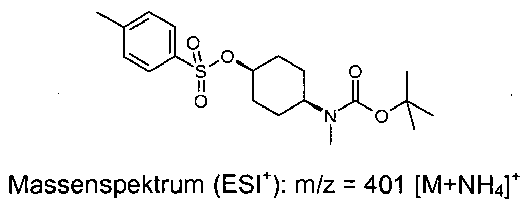 |
| IV(15) | 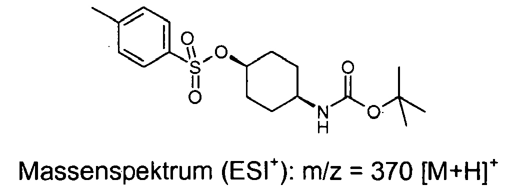 |
| IV(16) | 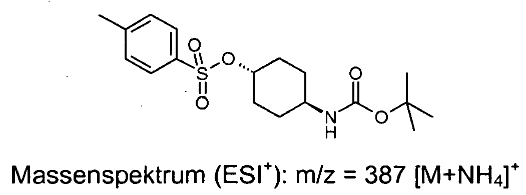 |

### Beispiel V

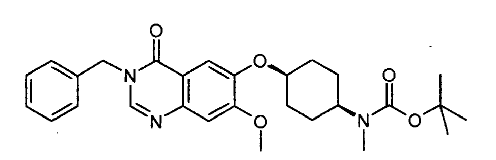

### 3-Benzyl-3,4-dihydro-4-oxo-6-{cis-4-[N-(tert-butyloxycarbonyl)-N-methyl-amino]-cyclohexyl-oxy}-7-methoxy-chinazolin

Hergestellt durch Umsetzung von 4 g 3-Benzyl-3,4-dihydro-4-oxo-6-hydroxy-7-methoxy-chinazolin und 8.77 g cis-1-Methansulfonyloxy-4-[N-(tert-butyloxycarbonyl)-N-methyl-amino]-cyclohexan in Gegenwart von 4.33 g Kaliumcarbonat in 32 ml N-Methyl-pyrrolidinon bei 100-120°C.
R_{f}-Wert: 0.78 (Kieselgel; Essigester/Methanol = 95:5)
Massenspektrum (ESI⁺): m/z = 494 [M+H]⁺

**Analog Beispiel V können erhalten werden:**

| **Beispiel** | **Struktur** |
|---|---|
| V(1) | 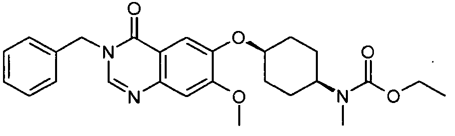 |
| V(2) | 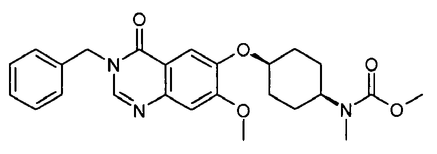 |
| V(3) | 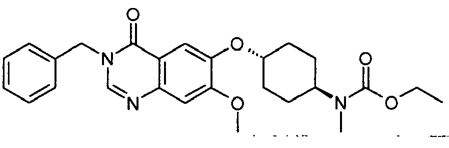 |
| V(4) | 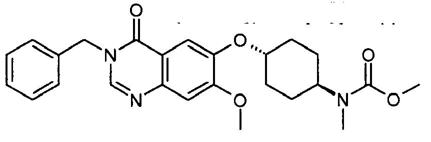 |
| V(5) | 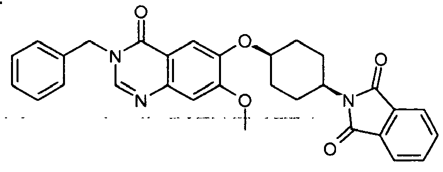 |
| V(6) | 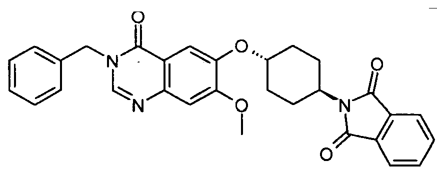 |
| V(7) | 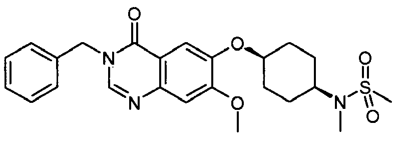 |
| V(8) | 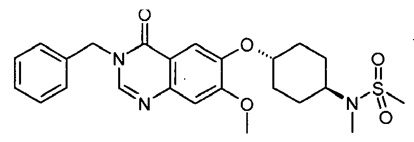 |
| V(9) | 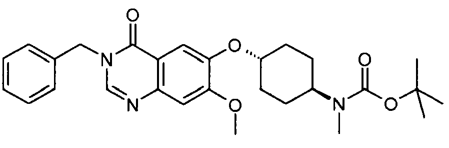 |
| V(10) | 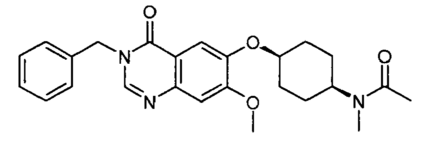 |
| V(11) | 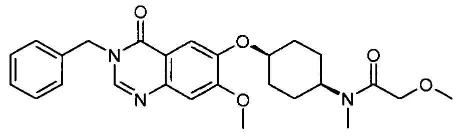 |
| V(12) | 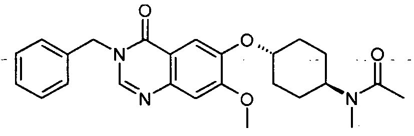 |
| V(13) | 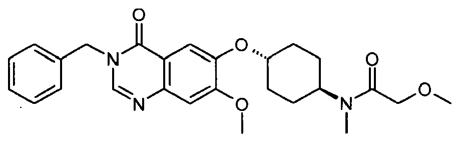 |

### Beispiel VI

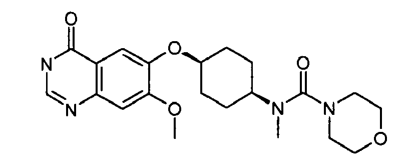

### 3,4-Dihydro-4-oxo-6-[cis-4-(N-morpholinocarbonyl-N-methyl-amino)-cyclohexyl-oxy]-7-methoxy-chinazolin

Eine Mischung aus 1.4 g 3-Benzyl-3,4-dihydro-4-oxo-6-[cis-4-(N-morpholinocarbonyl-N-methyl-amino)-cyclohexyl-oxy]-7-methoxy-chinazolin und 20 ml Eisessig werden in Gegenwart von 0.3 g Palladium auf Aktivkohle (10% Pd) bei 80°C und einem Wasserstoffdruck von 50 psi bis zur vollständigen Umsetzung hydriert. Der Katalysator wird abgesaugt, das Filtrat zur Trockene eingeengt und mit 15 ml Essigester versetzt. Der Niederschlag wird abgesaugt, mit 5 ml Essigester nachgewaschen und getrocknet. Ausbeute: 0.8 g (70% der Theorie)
R_{f}-Wert: 0.40 (Kieselgel, Methylenchlorid/Methanol = 9:1)
Massenspektrum (ESI⁺): m/z = 417 [M+H]⁺

Analog Beispiel VI können erhalten werden:

| **Beispiel** | **Struktur** |
|---|---|
| VI(1) | 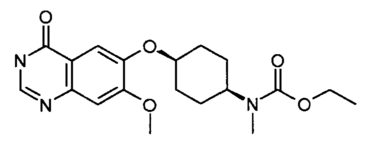 |
| VI(2) | 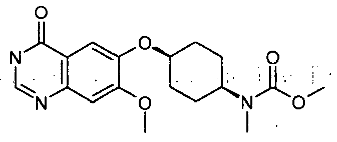 |
| VI(3) | 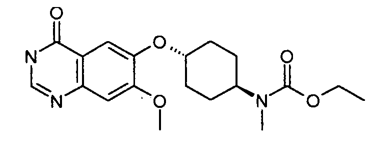 |
| VI(4) | 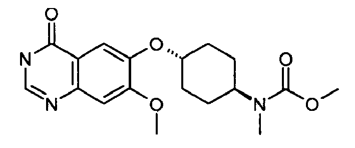 |
| VI(5) | 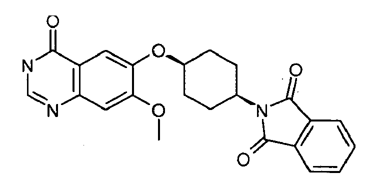 |
| VI(6) | 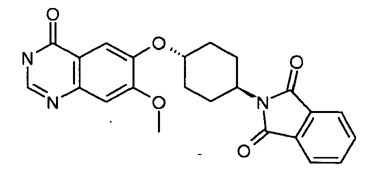 |
| VI(7) | 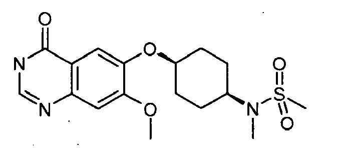 |
| VI(8) | 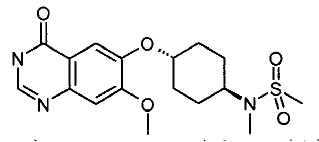 |
| VI(9) | 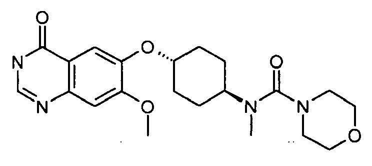 |
| VI(10) | 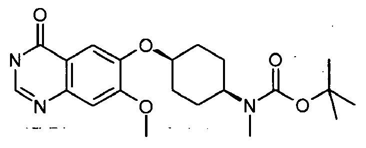 |
| VI(11) | 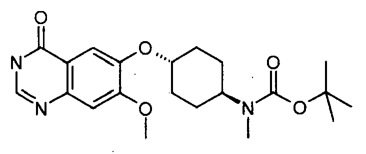 |
| VI(12) | 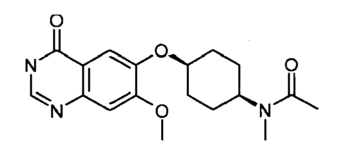 |
| VI(13) | 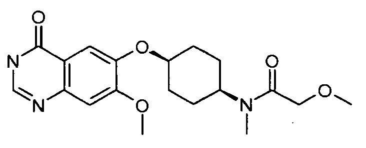 |
| VI(14) | 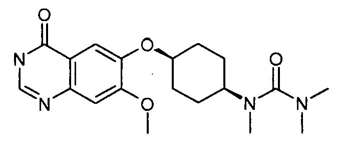 |
| VI(15) | 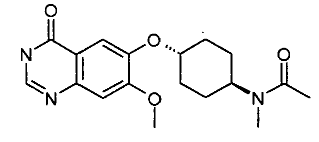 |
| VI(16) | 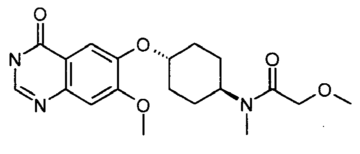 |
| VI(17) | 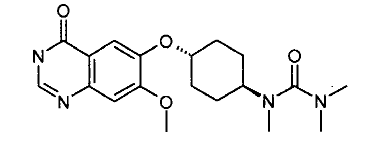 |

### Beispiel VII

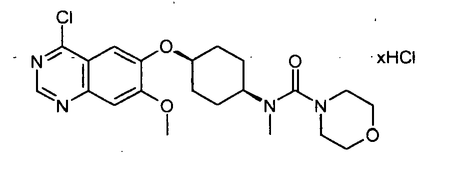

### 4-Chlor-6-[cis-4-(N-morphol inocarbonyl-N-methyl-amino)-cyclohexyl-oxy]-7-methoxy-chinazolin-hydrochlorid

800 mg 3,4-Dihydro-4-oxo-6-[cis-4-(N-morpholinocarbonyl-N-methyl-amino)-cyclohexyl-oxy]-7-methoxy-chinazolin, 7 ml Thionylchlorid und 0.1 ml Dimethylformamid werden für 3 Stunden unter Rückfluss erhitzt. Die flüchtigen Anteile des Reaktionsgemisches werden am Rotationsverdampfer abgezogen, der Rückstand mit Toluol versetzt und es wird erneut einrotiert.
Massenspektrum (ESI⁺): m/z = 435, 437 [M+H]⁺
Durch alkalische Aufarbeitung kann auch die freie Base erhalten werden.

Analog Beispiel VII können erhalten werden:

| **Beispiel** | **Struktur** |
|---|---|
| VII(1) | 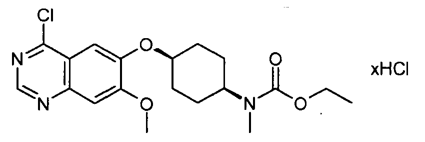 |
| VII(2) | 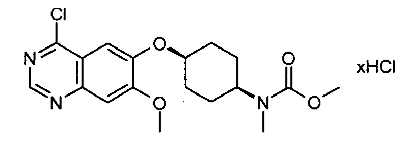 |
| VII(3) | 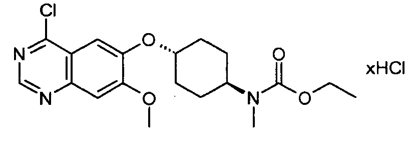 |
| VII(4) | 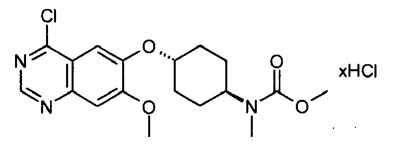 |
| VII(5) | 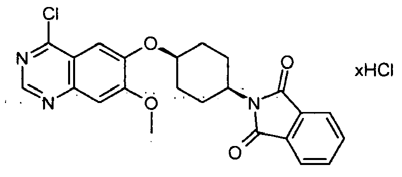 |
| VII(6) | 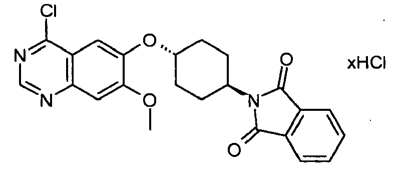 |
| VII(7) | 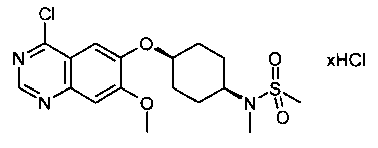 |
| VII(8) | 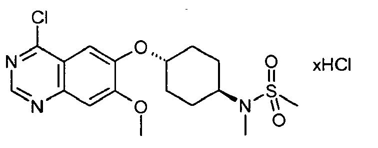 |
| VII(9) | 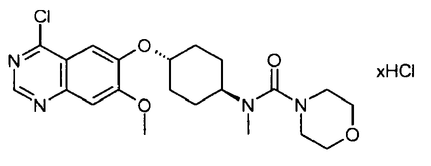 |
| VII(10) | 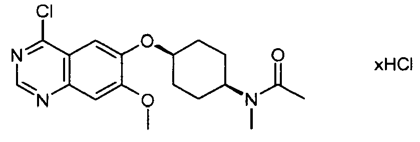 |
| VII(11) | 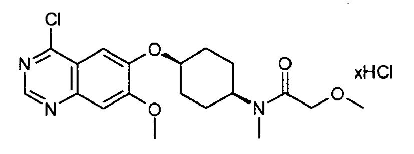 |
| VII(12) | 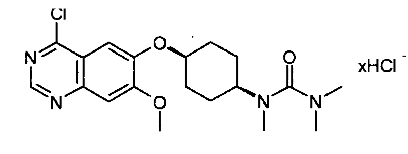 |
| VII(13) | 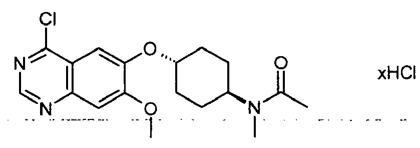 |
| VII(14) | 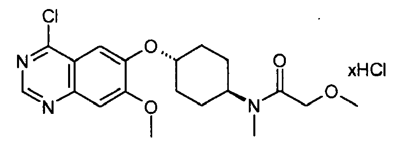 |
| VII(15) | 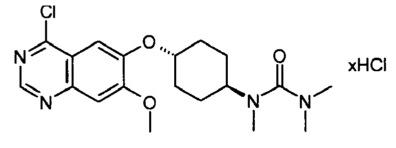 |

Durch alkalische Aufarbeitung können auch die freien Basen der vorstehend erwähnten Verbindungen erhalten werden.

### Beispiel VIII

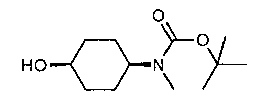

### cis-1-Hydroxy-4-(N-tert-butyloxycarbonyl-N-methyl-amino)-cyclohexan

Hergestellt durch Umsetzung von cis-1-Hydroxy-4-methylamino-cyclohexan mit Pyrokohlensäure-di-tert-butylester in Essigester bei Raumtemperatur.
Massenspektrum (ESI⁺): m/z = 230 [M+H]⁺

Analog Beispiel VIII können erhalten werden:

| **Beispiel** | **Struktur** |
|---|---|
| VIII(1) | 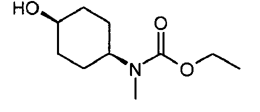 |
| VIII(2) | 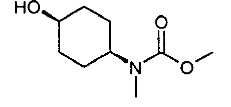 |
| VIII(3) | 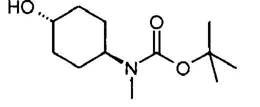 |
| VIII(4) | 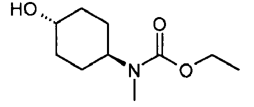 |
| VIII(5) | 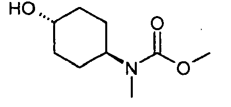 |
| VIII(6) | 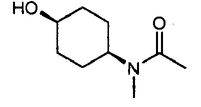 |
| VIII(7) | 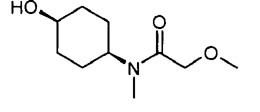 |
| VIII(8) | 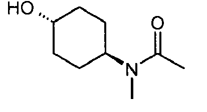 |
| VIII(9) | 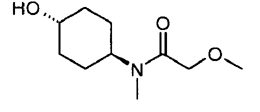 |

### Beispiel IX

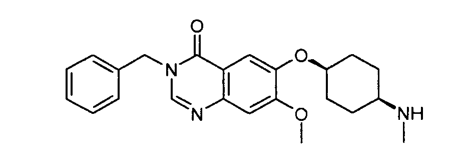

### 3-Benzyl-3,4-dihydro-4-oxo-6-(cis-4-methylamino-cyclohexan-1-yloxy)-7-methoxy-chinazolin-Hydrochlorid

Hergestellt durch Behandlung von 3-Benzyl-3,4-dihydro-4-oxo-6-{cis-4-[N-(tert-butyloxycarbonyl)-N-methyl-amino]-cyclohexyl-oxy}-7-methoxy-chinazolin mit isopropanolischer Salzsäure in Ethanol bei 40°C.
Massenspektrum (ESI⁺): m/z = 394 [M+H]⁺

Analog Beispiel IX können erhalten werden:

| **Beispiel** | **Struktur** |
|---|---|
| IX(1) | 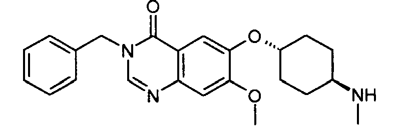 |
| IX(2) | 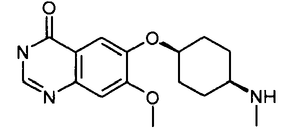 |
| IX(3) | 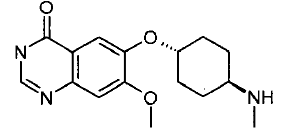 |

### Beispiel X

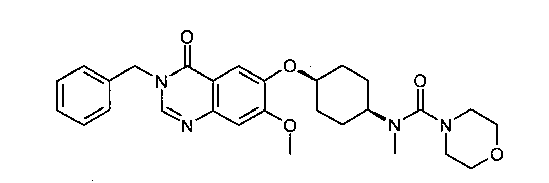

### 3-Benzyl-3,4-dihydro-4-oxo-6-[cis-4-(N-morpholinocarbonyl-N-methyl-amino)-cyclohexyl-oxy]-7-methoxy-chinazolin

Zu einem Gemisch aus 3 g 3-Benzyl-3,4-dihydro-4-oxo-6-(cis-4-methylamino-cyclohexyl-oxy)-7-methoxy-chinazolin-Hydrochlorid, 2.67 ml N-Ethyl-diisopropylamin und 25 ml Acetonitril werden unter Kühlung im Eisbad 1.18 ml Morpholinocarbonylchlorid gelöst in 5 ml Acetonitril zugetropft. Nach Rühren bei Raumtemperatur über Nacht wird das Reaktionsgemisch zwischen 50 ml Wasser und 30 ml Essigester verteilt. Die Wasserphase wird mit 50 ml Essigester extrahiert und die vereinigten organischen Phasen mit 20 ml Wasser und gesättigter Kochsalzlösung gewaschen, getrocknet und eingeengt. Der Rückstand wird durch Chromatographie über eine Kieselgelsäule mit Methylenchlorid/Methanol (97:3 bis 95:5) gereinigt.
Ausbeute: 1.5 g (42% der Theorie)
R_{f}-Wert: 0.60 (Kieselgel; Methylenchlorid/Methanol = 9:1)
Massenspektrum (ESI⁺): m/z = 507 [M+H]⁺

Analog Beispiel X können erhalten werden:

| **Beispiel** | **Struktur** |
|---|---|
| X(1) | 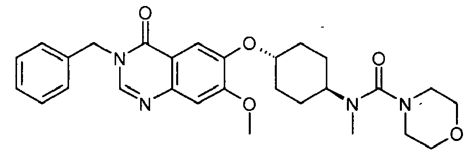 |
| X(2) | 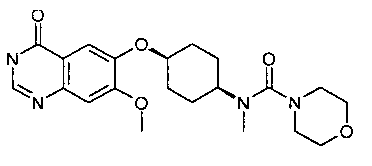 |
| X(3) | 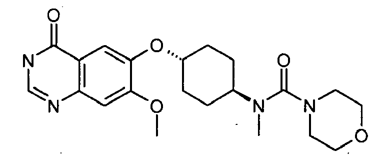 |
| X(4) | 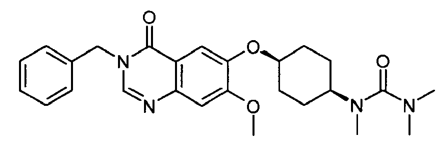 |
| X(5) | 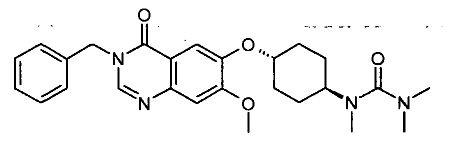 |
| X(6) | 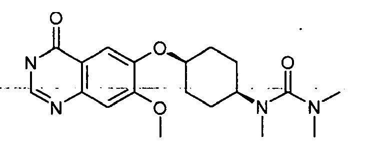 |
| X(7) | 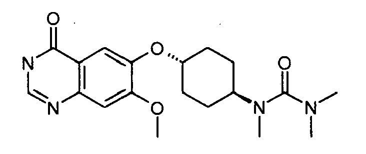 |
| X(8) | 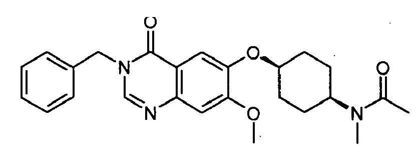 |
| X(9) | 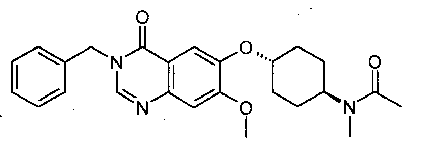 |
| X(10) | 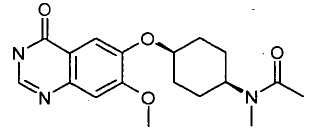 |
| X(11) | 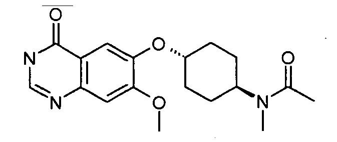 |
| X(12) | 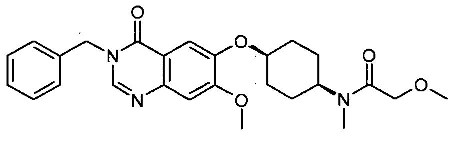 |
| X(13) | 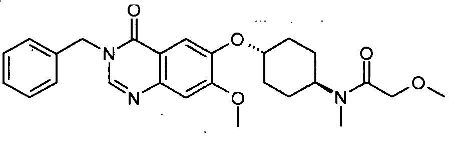 |
| X(14) | 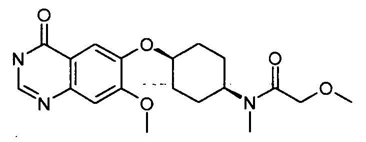 |
| X(15) | 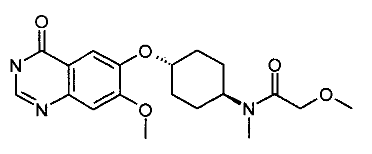 |

### Herstellung der Endverbindungen:

### Beispiel 1

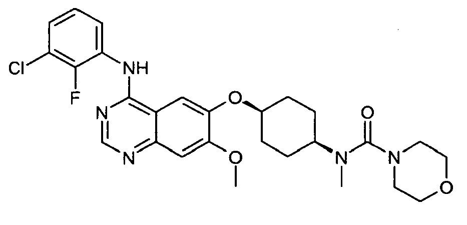

### 4-[(3-Chlor-2-fluor-phenyl)amino]-6-[cis-4-(N-morpholinocarbonyl-N-methyl-amino)-cyclohexyl-oxy]-7-methoxy-chinazolin

800 mg 3,4-Dihydro-4-oxo-6-[cis-4-(N-morpholinocarbonyl-N-methyl-amino)-cyclohexyl-oxy]-7-methoxy-chinazolin, 7 ml Thionylchlorid und 0.1 ml Dimethylformamid werden für 3 Stunden unter Rückfluss erhitzt. Die flüchtigen Anteile des Reaktionsgemisches werden am Rotationsverdampfer abgezogen, der Rückstand mit Toluol versetzt und es wird erneut einrotiert. Der Rückstand wird mit 30 ml Isopropanol und 643 mg 3-Chlor-2-fluor-anilin versetzt. Die Mischung wird 1.5 Stunden unter Rückfluss erhitzt. Danach wird zur Trockene eingeengt und der Rückstand zwischen 70 ml Essigester und 30 ml 10%iger wässriger Kaliumcarbonatlösung verteilt. Die organische Phase wird mit Wasser und Kochsalzlösung gewaschen, getrocknet und eingeengt. Der Rückstand wird durch Chromatographie über eine Kieselgelsäule mit Methylenchlorid/Methanol (95:5 bis 70:30) gereinigt.
Ausbeute: 580 mg (56% der Theorie)
R_{f}-Wert: 0.55-(Kieselgel; Methylenchlorid/Methanol = 7:1)
Massenspektrum (ESI⁺): m/z = 544, 546 [M+H]⁺

Analog Beispiel 1 können erhalten werden:

| **Beispiel 1** | **Struktur** |
|---|---|
| (1) | 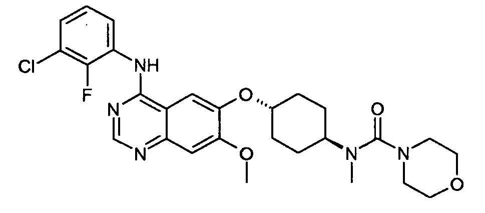 |
| | Massenspektrum (ESI⁺): m/z = 544, 546 [M+H]⁺ |
| (2) | 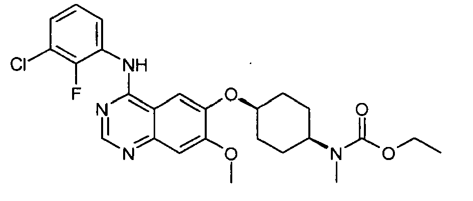 |
| (3) | 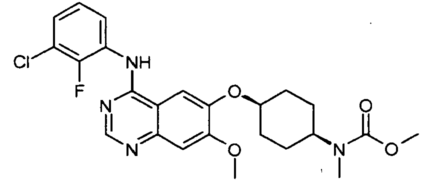 |
| (4) | 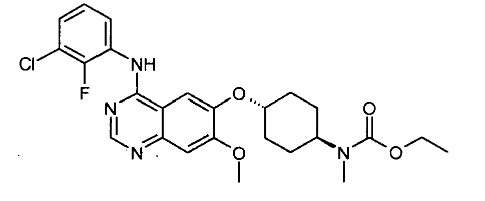 |
| (5) | 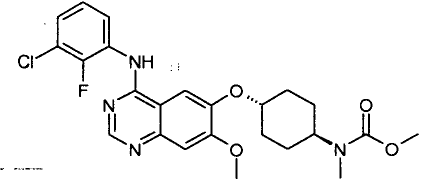 |
| (6) | 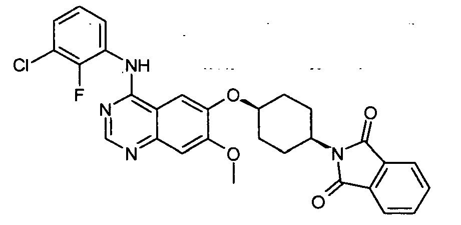 |
| (7) | 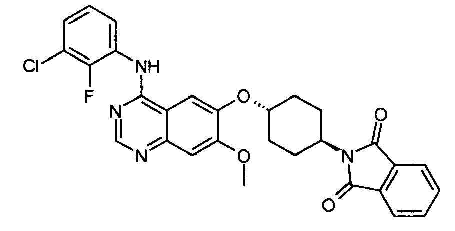 |
| (8) | 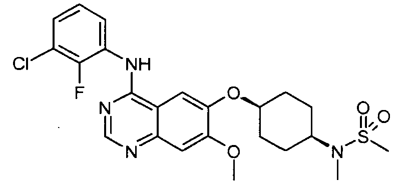 |
| (9) | 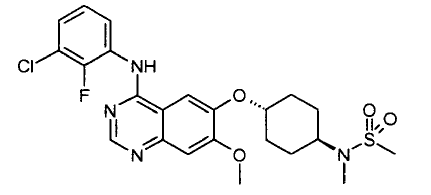 |
| (10) | 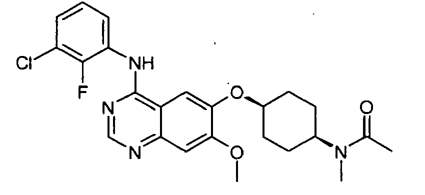 |
| (11) | 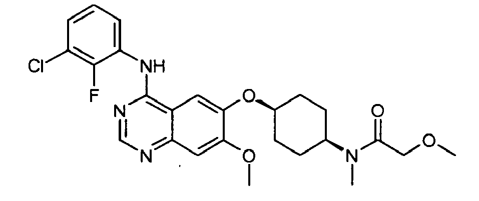 |
| (12) | 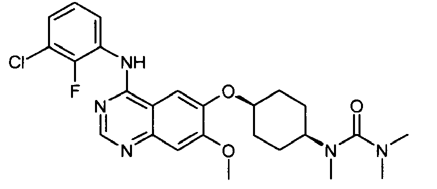 |
| (13) | 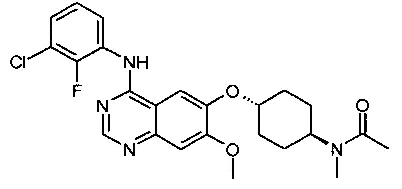 |
| (14) | 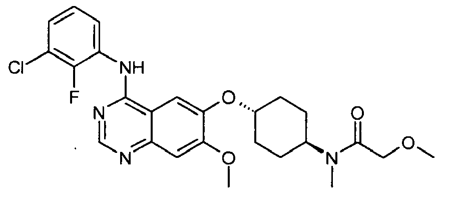 |
| (15) | 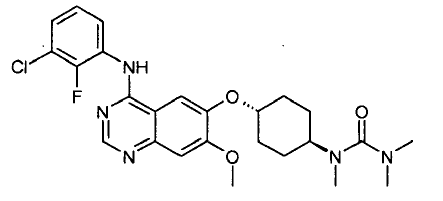 |

Analog den vorstehend genannten Beispielen und anderen literaturbekannten Verfahren können auch folgende Verbindungen hergestellt werden:

| **Beispiel 1** | **Struktur** |
|---|---|
| (16) | 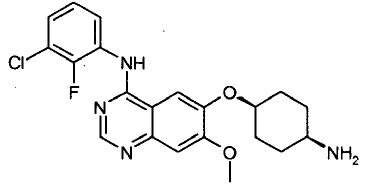 |
| (17) | 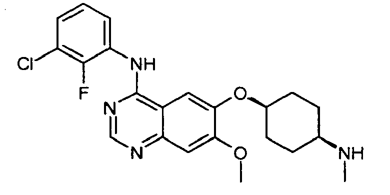 |
| (18) | 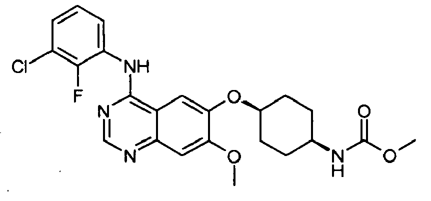 |
| (19) | 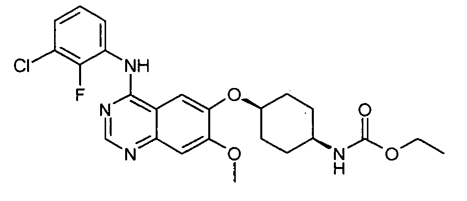 |
| (20) | 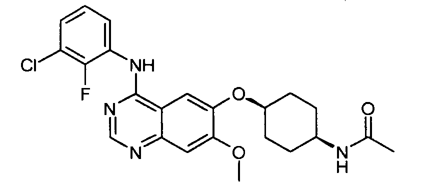 |
| (21) | 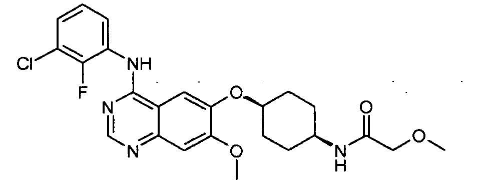 |
| (22) | 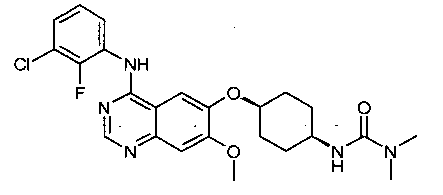 |
| (23) | 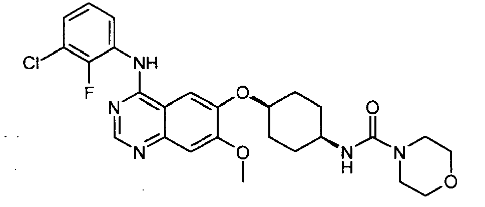 |
| | Massenspektrum (ESI⁺): m/z = 530, 532 [M+H]⁺ |
| (24) | 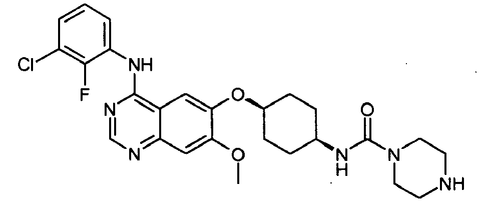 |
| (25) | 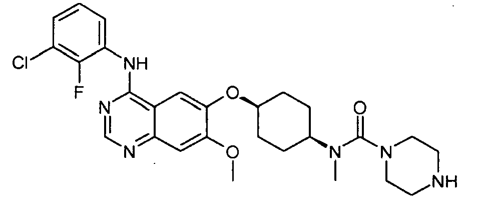 |
| (26) | 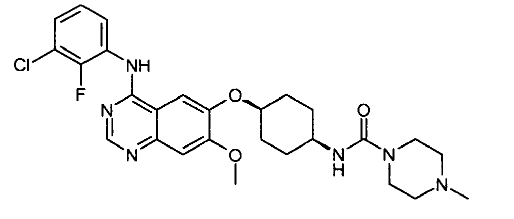 |
| (27) | 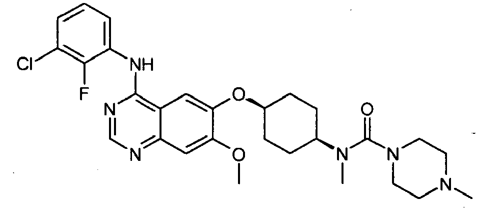 |
| (28) | 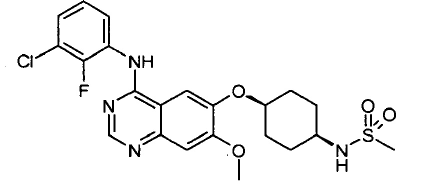 |
| (29) | 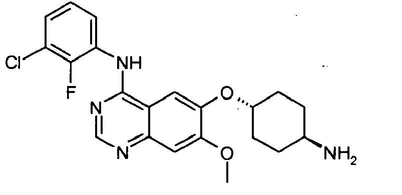 |
| (30) | 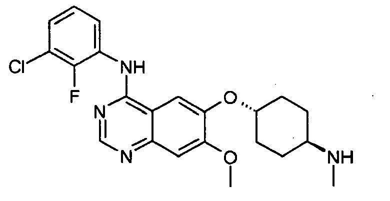 |
| (31) | 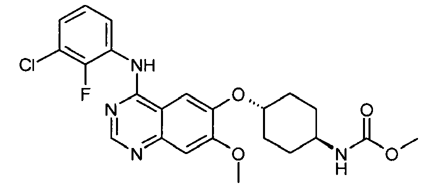 |
| (32) | 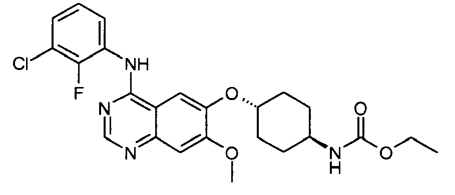 |
| (33) | 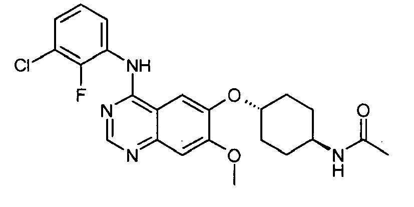 |
| (34) | 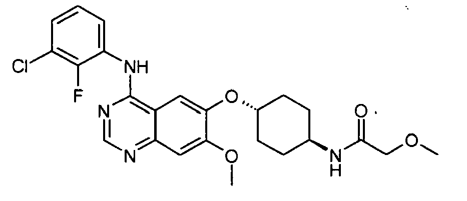 |
| (35) | 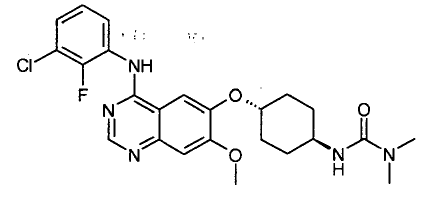 |
| (36) | 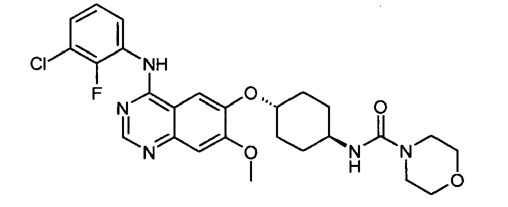 |
| | Massenspektrum (ESI⁺): m/z = 530, 532 [M+H]⁺ |
| (37) | 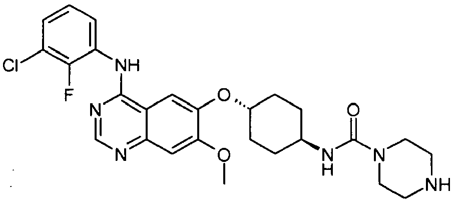 |
| (38) | 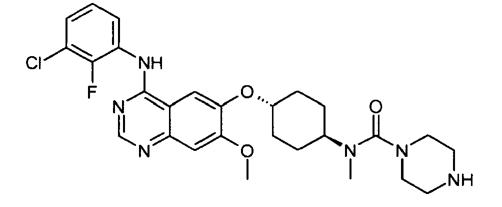 |
| (39) | 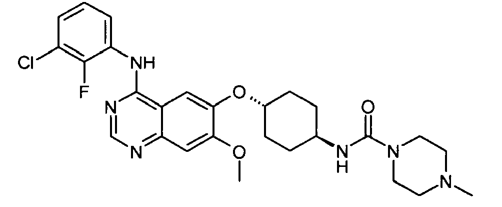 |
| (40) | 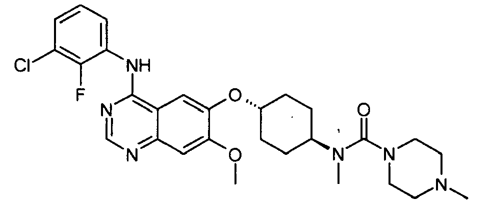 |
| (41) | 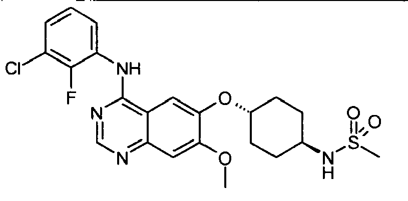 |

### Beispiel 2

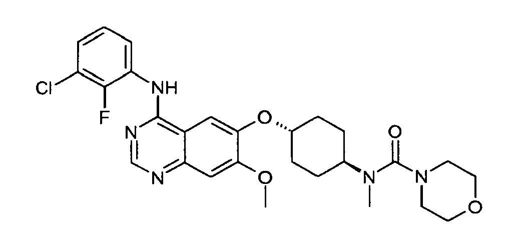

### 4-[(3-Chlor-2-fluor-phenyl)amino]-6-[trans-4-(N-morpholinocarbonyl-N-methyl-amino)-cyclohexyl-oxy]-7-methoxy-chinazol in

2600 mg 4-[(3-Chlor-2-fluor-phenyl)amino]-6-[trans-4-(methylamino)-cyclohexyl-oxy]-7-methoxy-chinazolin-hydrochlorid werden in 20 ml Acetonitril suspendiert, dann werden 3.1 ml Triethylamin zugesetzt und 0.723 ml Morpholin-N-carbonylchlorid, gelöst in 5 ml Acetonitril, bei < 8°C zugetropft. Nach Rühren bei Raumtemperatur über Nacht wird das Reaktionsgemisch mit Essigester verdünnt und die organische Phase mit Wasser und Kochsalzlösung ausgeschüttelt. Die organische Phase wird getrocknet und eingeengt. Der Rückstand wird mit Acetonitril ausgerührt, der Feststoff abgesaugt und getrocknet.
Massenspektrum (ESI⁺): m/z = 544, 546 [M+H]⁺

Analog Beispiel 2 werden erhalten:

| **Beispiel 2** | **Struktur** |
|---|---|
| (1) | 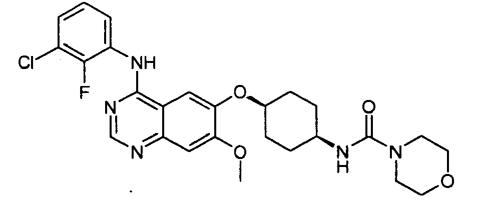 |
| | Massenspektrum (ESI⁺): m/z = 530, 532 [M+H]⁺ |
| (2) | 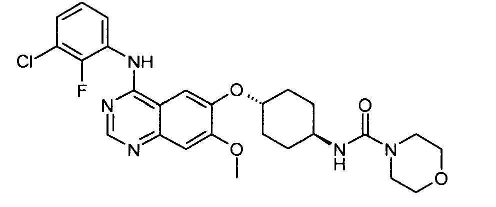 |
| | Massenspektrum (ESI⁺): m/z = 530, 532 [M+H]⁺ |

### Beispiel 3

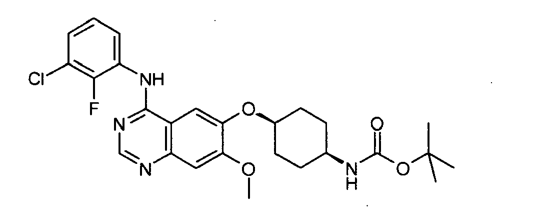

### 4-[(3-Chlor-2-fluor-phenyl)amino]-6-[cis-4-(tert.-butoxycarbonyl-amino)-cyclohexyl-oxy]-7-methoxy-chinazolin

Hergestellt durch Umsetzung von 5 g 4-[(3-Chlor-2-fluor-phenyl)amino]-6-hydroxy-7-methoxy-chinazolin und 8.7 g der Verbindung des Beispiels IV(16) in Gegenwart von 4.3 g Kaliumcarbonat in 40 ml N,N-Dimethylformamid bei 80°C.
Massenspektrum (ESI⁺): m/z = 517, 519 [M+H]⁺

Analog Beispiel 3 werden erhalten:

| **Beispiel 3** | **Struktur** |
|---|---|
| (1) | 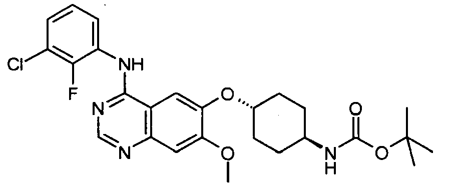 |
| | Massenspektrum (ESI⁺): m/z = 517, 519 [M+H]⁺ |
| (2) | 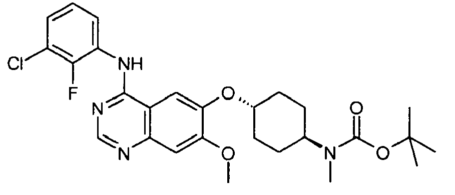 |
| | Massenspektrum (ESI⁺): m/z = 531, 533 [M+H]⁺ |
| (3) | 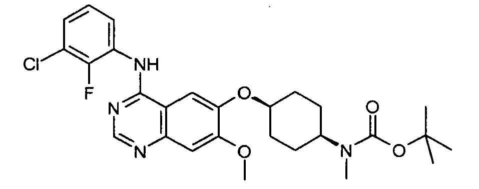 |
| | Massenspektrum (ESI⁺): m/z = 531, 533 [M+H]⁺ |

### Beispiel 4

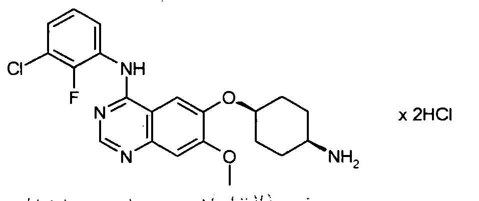

### 4-[(3-Chlor-2-fluor-phenyl)amino]-6-[cis-4-(amino)-cyclohexyl-oxy]-7-methoxy-chinazolin-dihydrochlorid

Hergestellt durch Behandlung von 843 mg der Verbindung des Beispiels 3 mit 3.3 ml isopropanolischer Salzsäure (5-6 M) in 8 ml Dichlormethan bei Raumtemperatur.
Massenspektrum (ESI⁺): m/z = 417, 419 [M+H]⁺

Analog Beispiel 4 werden folgende Verbindungen erhalten:

| **Beispiel 4** Beispiel 4 | **Struktur** |
|---|---|
| (1) | 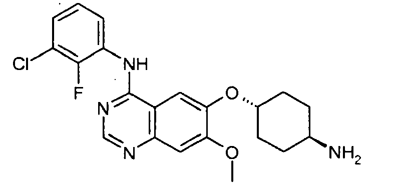 |
| | Massenspektrum (ESI⁺): m/z = 417, 419 [M+H]⁺ |
| (2) | 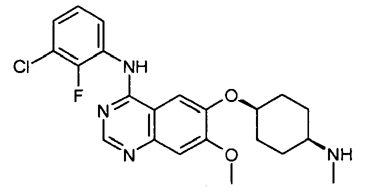 |
| | Massenspektrum (ESI⁺): m/z = 431, 433 [M+H]⁺ |
| (3) | 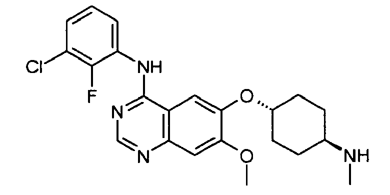 |
| | Massenspektrum (ESI⁺): m/z = 431, 433 [M+H]⁺ |

### Beispiel 5

### Dragees mit 75 mg Wirksubstanz

### Zusammensetzung:

| | | |
|---|---|---|
| 1 | Dragéekern enthält: | |
| | Wirksubstanz | 75.0 mg |
| | Calciumphosphat | 93.0 mg |
| | Maisstärke | 35.5 mg |
| | Polyvinylpyrrolidon | 10.0 mg |
| | Hydroxypropylmethylcellulose | 15.0 mg |
| | Magnesiumstearat | 1.5 mg |
| | | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | | |
|---|---|---|
| Kerngewicht: | 230 | mg |
| Stempel: | 9 | mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht: | 245 mg. |

### Beispiel 6

### Tabletten mit 100 mg Wirksubstanz

### Zusammensetzung:

| | | |
|---|---|---|
| 1 | Tablette enthält: | |
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | | |
|---|---|---|
| Tablettengewicht: | 220 mg | |
| Durchmesser: | 10 mm, | biplan mit beidseitiger Facette und einseitiger Teilkerbe. |

### Beispiel 7

### Tabletten mit 150 mg Wirksubstanz

### Zusammensetzung:

| | | |
|---|---|---|
| 1 | Tablette enthält: | |
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | | |
|---|---|---|
| Tablettengewicht: | 300 | mg |
| Stempel: | 10 | mm, flach |

### Beispiel 8

### Hartgelatine-Kapseln mit 150 mg Wirksubstanz

### Zusammensetzung:

| | | |
|---|---|---|
| 1 | Kapsel enthält: | |
| | Wirkstoff | 150.0 mg |
| | Maisstärke getr. ca. | 180.0 mg |
| | Milchzucker pulv. ca. | 87.0 mg |
| | Magnesiumstearat | 3.0 mg |
| | ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 9

### Suppositorien mit 150 mg Wirksubstanz

### Zusammensetzung:

| | | |
|---|---|---|
| 1 | Zäpfchen enthält: | |
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel 10

### Suspension mit 50 mg Wirksubstanz

### Zusammensetzung:

| | | |
|---|---|---|
| 100 ml | Suspension enthalten: | |
| | Wirkstoff | 1.00 g |
| | Carboxymethylcellulose-Na-Salz | 0.10 g |
| | p-Hydroxybenzoesäuremethylester | 0.05 g |
| | p-Hydroxybenzoesäurepropylester | 0.01 g |
| | Rohrzucker | 10.00 g |
| | Glycerin | 5.00 g |
| | Sorbitlösung 70%ig | 20.00 g |
| | Aroma | 0.30 g |
| | Wasser dest.ad 100.00 ml | |

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 11

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel 12

### Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### Beispiel 13

### Kapseln zur Pulverinhalation mit 5 mg Wirksubstanz

### 1 Kapsel enthält:

| | |
|---|---|
| Wirksubstanz | 5.0 mg |
| Lactose für Inhalationszwecke | 15.0 mg |
| | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

### Beispiel 14

### Inhalationslösung für Handvernebler mit 2.5 mg Wirksubstanz

### 1 Hub enthält:

| | | |
|---|---|---|
| Wirksubstanz | | 2.500 mg |
| Benzalkoniumchlorid | | 0.001 mg |
| 1 N-Salzsäure q.s. | | |
| Ethanol/Wasser (50/50) | ad | 15.000 mg |

### Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.
Füllmasse des Behälters: 4.5 g

## Patentansprüche

1. Bicyclische Heterocyclen der allgemeinen Formel (I) 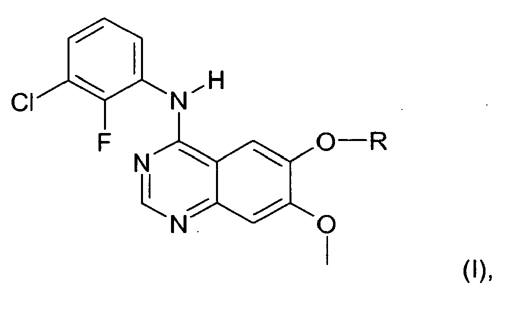 worin
R ein Rest ausgewählt aus der Gruppe bestehend aus
cis-4-Amino-cyclohexyl, trans-4-Amino-cyclohexyl,
cis-4-Methylamino-cyclohexyl, trans-4-Methylamino-cyclohexyl,
cis-4-(Methoxycarbonylamino)-cyclohexyl, trans-4-(Methoxycarbonylamino)-cyclohexyl,
cis-4-(N-Methoxycarbonyl-N-methyl-amino)-cyclohexyl-, trans-4-(N-Methoxycarbonyl-N-methyl-amino)-cyclohexyl,
cis-4-(Ethyloxycarbonylamino)-cyclohexyl, trans-4-(Ethyloxycarbonylamino)-cyclohexyl,
cis-4-(N-Ethyloxycarbonyl-N-methyl-amino)-cyclohexyl, trans-4-(N-Ethyloxycarbonyl-N-methyl-amino)-cyclohexyl,
cis-4-(tert.-Butoxycarbonylamino)-cyclohexyl, trans-4-(tert.-Butoxycarbonylamino)-cyclohexyl,
cis-4-(N-tert.-Butoxycarbonyl-N-methyl-amino)-cyclohexyl, trans-4-(N-tert.-Butoxycarbonyl-N-methyl-amino)-cyclohexyl,
cis-4-(Acetylamino)-cyclohexyl, trans-4-(Acetylamino)-cyclohexyl,
cis-4-(N-Acetyl-N-methyl-amino)-cyclohexyl, trans-4-(N-Acetyl-N-methyl-amino)-cyclohexyl,
cis-4-(Methoxyacetyl-amino)-cyclohexyl, trans-4-(Methoxyacetyl-amino)-cyclohexyl,
cis-4-(N-Methoxyacetyl-N-methyl-amino)-cyclohexyl, trans-4-(N-Methoxyacetyl-N-methyl-amino)-cyclohexyl,
cis-4-(Dimethylaminocarbonyl-amino)-cyclohexyl, trans-4-(Dimethylaminocarbonyl-amino)-cyclohexyl,
cis-4-(N-Dimethylaminocarbonyl-N-methyl-amino)-cyclohexyl, trans-4-(N-Dimethylaminocarbonyl-N-methyl-amino)-cyclohexyl,
cis-4-(Morpholinocarbonyl-amino)-cyclohexyl, trans-4-(Morpholinocarbonyl-amino)-cyclohexyl,
cis-4-(N-Morpholinocarbonyl-N-methyl-amino)-cyclohexyl, trans-4-(N-Morpholinocarbonyl-N-methyl-amino)-cyclohexyl,
cis-4-(Piperazin-1-ylcarbonyl-amino)-cyclohexyl, trans-4-(Piperazin-1-ylcarbonyl-amino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl,
cis-4-[(4-Methyl-piperazin-1-ylcarbonyl)-amino]-cyclohexyl, trans-4-[(4-Methylpiperazin-1-ylcarbonyl)-amino]-cyclohexyl,
cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl,
cis-4-(Methansulfonylamino)-cyclohexyl, trans-4-(Methansulfonylamino)-cyctohexyl,
cis-4-(N-Methansulfonyl-N-methyl-amino)-cyclohexyl, trans-4-(N-Methansulfonyl-N-methyl-amino)-cyclohexyl, cis-4-Phthalimido-cyclohexyl und trans-4-Phthalimido-cyclohexyl,
gegebenenfalls in Form ihrer Tautomeren und ihrer Gemische, sowie gegebenenfalls ihrer pharmakologisch unbedenklichen Säureadditionssalze, Solvate und Hydrate,
bedeutet.

2. Verbindungen nach Anspruch 1,
R ein Rest ausgewählt aus der Gruppe bestehend aus
cis-4-(Methoxycarbonylamino)-cyclohexyl, trans-4-(Methoxycarbonylamino)-cyclohexyl,
cis-4-(N-Methoxycarbonyl-N-methyl-amino)-cyclohexyl-, trans-4-(N-Methoxycarbonyl-N-methyl-amino)-cyclohexyl,
cis-4-(Ethyloxycarbonylamino)-cyclohexyl, trans-4-(Ethyloxycarbonylamino)-cyclohexyl,
cis-4-(N-Ethyloxycarbonyl-N-methyl-amino)-cyclohexyl, trans-4-(N-Ethyloxycarbonyl-N-methyl-amino)-cyclohexyl,
cis-4-(tert.-Butoxycarbonylamino)-cyclohexyl, trans-4-(tert.-Butoxycarbonylamino)-cyclohexyl,
cis-4-(N-tert.-Butoxycarbonyl-N-methyl-amino)-cyclohexyl, trans-4-(N-tert.-Butoxycarbonyl-N-methyl-amino)-cyclohexyl,
cis-4-(Methoxyacetyl-amino)-cyclohexyl, trans-4-(Methoxyacetyl-amino)-cyclohexyl,
cis-4-(N-Methoxyacetyl-N-methyl-amino)-cyclohexyl, trans-4-(N-Methoxyacetyl-N-methyl-amino)-cyclohexyl,
cis-4-(Dimethylaminocarbonyl-amino)-cyclohexyl, trans-4-(Dimethylaminocarbonyl-amino)-cyclohexyl,
cis-4-(N-Dimethylaminocarbonyl-N-methyl-amino)-cyclohexyl, trans-4-(N-Dimethylaminocarbonyl-N-methyl-amino)-cyclohexyl,
cis-4-(Morpholinocarbonyl-amino)-cyclohexyl, trans-4-(Morpholinocarbonyl-amino)-cyclohexyl,
cis-4-(N-Morpholinocarbonyl-N-methyl-amino)-cyclohexyl, trans-4-(N-Morpholinocarbonyl-N-methyl-amino)-cyclohexyl,
cis-4-(Piperazin-1-ylcarbonyl-amino)-cyclohexyl, trans-4-(Piperazin-1-ylcarbonyl-amino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl,
cis-4-[(4-Methyl-piperazin-1-ylcarbonyl)-amino]-cyclohexyl, trans-4-[(4-Methylpiperazin-1-ylcarbonyl)-amino]-cyclohexyl,
cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl,
cis-4-(Methansulfonylamino)-cyclohexyl, trans-4-(Methansulfonylamino)-cyclohexyl,
cis-4-(N-Methansulfonyl-N-methyl-amino)-cyclohexyl, trans-4-(N-Methansulfonyl-N-methyl-amino)-cyclohexyl, cis-4-Phthalimido-cyclohexyl und trans-4-Phthalimido-cyclohexyl,
gegebenenfalls in Form ihrer Tautomeren und ihrer Gemische, sowie gegebenenfalls ihrer pharmakologisch unbedenklichen Säureadditionssalze, Solvate und Hydrate,
bedeutet.

3. Physiologisch verträgliche Salze der Verbindungen gemäß Anspruch 1 mit anorganischen oder organischen Säuren.

4. Arzneimittel, enthaltend eine Verbindung gemäß Anspruch 1 oder ein physiologisch verträgliches Salz gemäß Anspruch 2 neben gegebenenfalls einem oder mehreren inerten Trägerstoffen und/oder Verdünnungsmitteln.

5. Verwendung einer Verbindung gemäß mindestens einem der Ansprüche 1 bis 2 zur Herstellung eines Arzneimittels, das zur Behandlung von benignen oder malignen Tumoren, zur Vorbeugung und Behandlung von Erkrankungen der Atemwege und der Lunge sowie zur Behandlung von Erkrankungen des Magen-Darm-Traktes und der Gallengänge und -blase geeignet ist.

6. Verfahren zur Herstellung der Verbindungen der allgemeinen Formel I gemäß Anspruch 1, **dadurch gekennzeichnet, dass**
a) eine Verbindung der Formel (II) 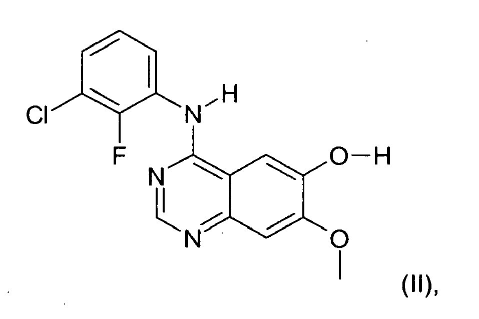 mit einer Verbindung der allgemeinen Formel (III)
Z¹ - R (III),
worin,
R wie im Anspruch 1 definiert ist, und
Z¹ eine Austrittsgruppe oder Hydroxygruppe darstellt, umgesetzt wird, oder
b) eine Verbindung der allgemeinen Formel (IV) 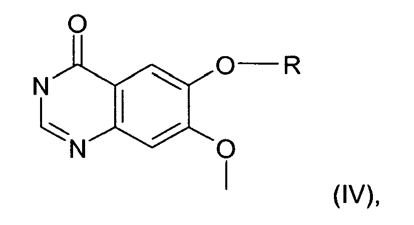 in der R wie im Anspruch 1 definiert ist,
mit einem Halogenierungsmittel zu einer Zwischenverbindung der allgemeinen Formel (V), 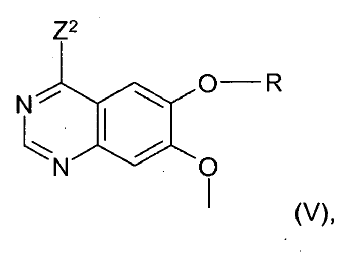 in der R wie im Anspruch 1 definiert ist, und Z² ein Halogenatom darstellt, umgesetzt wird, und anschließend mit 3-Chlor-2-fluor-anilin umgesetzt wird.

7. Verfahren zur Herstellung der Verbindungen der allgemeinen Formel (I) gemäß Anspruch 1, **dadurch gekennzeichnet, dass**
R ein Rest ausgewählt aus der Gruppe bestehend aus cis-4-(Methoxycarbonylamino)-cyclohexyl, trans-4-(Methoxycarbonylamino)-cyclohexyl, cis-4-(N-Methoxycarbonyl-N-methyl-amino)-cyclohexyl-, trans-4-(N-Methoxycarbonyl-N-methyl-amino)-cyclohexyl, cis-4-(Ethyloxycarbonylamino)-cyclohexyl, trans-4-(Ethyloxycarbonylamino)-cyclohexyl, cis-4-(N-Ethyloxycarbonyl-N-methyl-amino)-cyclohexyl, trans-4-(N-Ethyloxycarbonyl-N-methyl-amino)-cyclohexyl, cis-4-(tert.-Butoxycarbonylamino)-cyclohexyl, trans-4-(tert.-Butoxycarbonylamino)-cyclohexyl, cis-4-(N-tert.-Butoxycarbonyl-N-methyl-amino)-cyclohexyl, trans-4-(N-tert.-Butoxycarbonyl-N-methyl-amino)-cyclohexyl,
cis-4-(Acetylamino)-cyclohexyl, trans-4-(Acetylamino)-cyclohexyl,cis-4-(N-Acetyl-N-methyl-amino)-cyclohexyl, trans-4-(N-Acetyl-N-methyl-amino)-cyclohexyl, cis-4-(Methoxyacetyl-amino)-cyclohexyl, trans-4-(Methoxyacetyl-amino)-cyclohexyl,cis-4-(N-Methoxyacetyl-N-methyl-amino)-cyclohexyl, trans-4-(N-Methoxyacetyl-N-methyl-amino)-cyclohexyl, cis-4-(Dimethylaminocarbonyl-amino)-cyclohexyl, trans-4-(Dimethylaminocarbonyl-amino)-cyclohexyl,cis-4-(N-Dimethylaminocarbonyl-N-methyl-amino)-cyclohexyl, trans-4-(N-Dimethylaminocarbonyl-N-methyl-amino)-cyclohexyl, cis-4-(Morpholinocarbonyl-amino)-cyclohexyl, trans-4-(Morpholinocarbonyl-amino)-cyclohexyl,cis-4-(N-Morpholinocarbonyl-N-methyl-amino)-cyclohexyl, trans-4-(N-Morpholinocarbonyl-N-methyl-amino)-cyclohexyl, cis-4-(Piperazin-1-ylcarbonyl-amino)-cyclohexyl, trans-4-(Piperazin-1-ylcarbonyl-amino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl, cis-4-[(4-Methyl-piperazin-1-ylcarbonyl)-amino]-cyclohexyl, trans-4-[(4-Methyl-piperazin-1-ylcarbonyl)-amino]-cyclohexyl, cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl, cis-4-(Methansulfonylamino)-cyclohexyl, trans-4-(Methansulfonylamino)-cyclohexyl, cis-4-(N-Methansulfonyl-N-methyl-amino)-cyclohexyl und trans-4-(N-Methansulfonyl-N-methyl-amino)-cyclohexyl,
bedeutet,
eine Verbindung der allgemeinen Formel (VI) 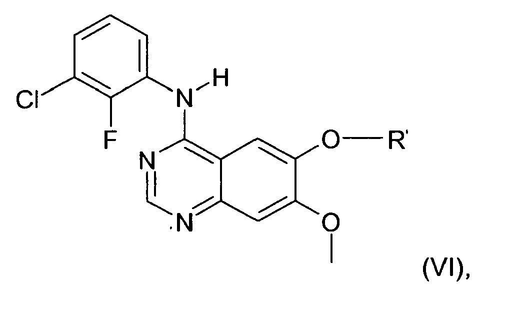 worin
R' ein Rest ausgewählt aus der Gruppe bestehend aus cis-4-Amino-cyclohex-1- yl, trans-4-Amino-cyclohex-1-yl, cis-4-(Methylamino)-cyclohex-1-yl und trans- 4-(Methylamino)-cyclohex-1-yl, darstellt,
mit einem entsprechenden Acylierungsmittel umgesetzt wird.

8. Verfahren zur Herstellung der Verbindungen der allgemeinen Formel (I) gemäß Anspruch 1 , **dadurch gekennzeichnet, dass**
R eine cis-4-Phthalimido-cyclohex-1-yl- oder trans-4-Phthalimido-cyclohex-1-yl- Gruppe bedeutet, und
eine Verbindung der allgemeinen Formel (VII) 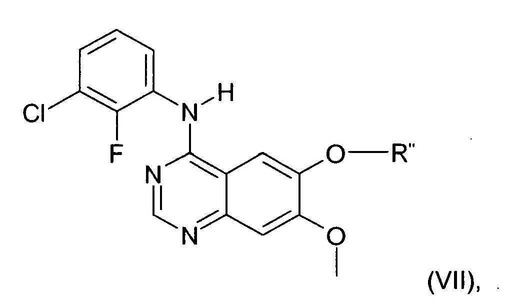 worin,
R" eine cis-4-Amino-cyclohex-1-yl- oder trans-4-Amino-cyclohex-1-yl-Gruppe darstellt,
mit Phthalsäureanhydrid oder einem anderen reaktiven Derivat der Phthalsäure ungesetzt wird.

9. Arzneimittelkombinationen, die neben einer oder mehrerer Verbindungen der Formel **1** gemäß einem der Ansprüche 1 oder 2 als weiteren Wirkstoff einen oder mehrere Verbindungen enthalten, die ausgewählt sind aus den Klassen der Betamimetika, Anticholinergika, Corticosteroiden, weitere PDE4-Inhibitoren, LTD4-Antagonisten, EGFR-Hemmern, Dopamin-Agonisten, H1-Antihistaminika, PAF-Antagonisten, P13-Kinase Inhibitoren, MPR4-Inhibitoren, iNOS-Inhibitoren und SYK-Inhibitoren oder zwei- oder dreifach Kombinationen davon.

## Claims

1. Bicyclic heterocycles of general formula (I) 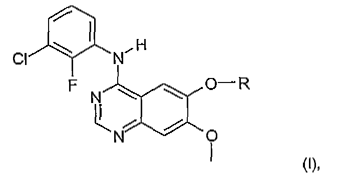 wherein
R denotes a group selected from among
cis-4-amino-cyclohexyl, trans-4-amino-cyclohexyl,
cis-4-methylamino-cyclohexyl, trans-4-methylamino-cyclohexyl,
cis-4-(methoxycarbonylamino)-cyclohexyl, trans-4-(methoxycarbonylamino)-cyclohexyl,
cis-4-(N-methoxycarbonyl-N-methyl-amino)-cyclohexyl, trans-4-(N-methoxycarbonyl-N-methyl-amino)-cyolohexyl,
cis-4-(ethyloxycarbonylamino)-cyclohexyl, trans-4-(ethyloxycarbonylamino)-cyclohexyl,
cis-4-(N-ethyloxycarbonyl-N-methyl-amino)-cyclohexyl, trans-4-(N-ethyloxycarbonyl-N-methyl-amino)-cyclohexyl,
cis-4-(tert.-butoxycarbonylamino)-cyclohexyl, trans-4-(tert.-butoxycarbonylamino)-cyclohexyl,
cis-4-(N-tert.-butoxycarbonyl-N-methyl-amino)-cyclohexyl, trans-4-(N-tert.-butoxycarbonyl-N-methyl-amino)-cyclohexyl,
cis-4-(acetylamino)-cyclohexyl, trans-4-(acetylamino)-cyclohexyl,
cis-4-(N-acetyl-N-methyl-amino)-cyrlohexyl, trans-4-(N-acetyl-N-methyl-amino)-cyclohexyl,
cis-4-(methoxyacetyl-amino)-cyclohexyl, trans-4-(methoxyacetyl-amino)-cyclohexyl,
cis-4-(N-methoxyacetyl-N-methyl-amino)-cyclohexyl, trans-4-(N-methoxyacetyl-N-methyl-amino)-cyclohexyl,
cis-4-(dimethylaminocarbonyl-amino)-cyclohexyl, trans-4-(dimethylaminocarbonyl-amino)-cyclohexyl,
cis-4-(N-dimethylaminocarbonyl-N-methyl-amino)-cyclohexyl, trans-4-(N-dimethylaminocarbonyl-N-methyl-amino)-cyclohexyl,
cis-4-(morpholinocarbonyl-amino)-cyclohexyl, trans-4-(morpholinocarbonyl-amino)-cyclohexyl,
cis-4-(N-morpholinocarbonyl-N-methyl-amino)-cyclohexyl, trans-4-(N-morpholinocarbonyl-N-methyl-amino)-cyclohexyl,
cis-4-(piperazin-1-ylcarbonyl-amino)-cyclohexyl, trans-4-(piperazin-1-ylcarbonyl-amino)-cyclohexyl,
cis-4-(N-piperazin-1-ylcarbonyl-N-methyl-amino)-cyclohexyl, trans-4-(N-piperazin-1-ylcarbonyl-N-mathyl-amino)-cyclohexyl,
cis-4-[(4-methyl-piperazin-1-ylcarbonyl)-amino]-cyclohexyl, trans-4-[(4-methyl-piperazin-1-ylcarbonyl)-amino]-cyclohexyl,
cis-A-[N-(4-methyl-piperazin-1-ylcarbonyl)-N-methyl-amino]-cyclohexyl, trans-4-[N-(4-methyl-piperazin-1-ylcarbanyl)-N-methyl-amino]-cyclohexyl,
cis-4-(methanesulphohylamino)-cydohexyl, trans-4-(methanesulphonylamino)-cyclohexyl,
cis-4-(N-methanesulphonyl-N-methyl-amino)-cyclohexyl, trans-4-(N-methanesulphonyl-N-methyl-amino)-cyclohexyl, cis-4-phthalimido-cydohexyl and trans-4-phthalimido-cyclohexyl,
optionally in the form of the tautomers and mixtures thereof, and optionally the pharmacologically acceptable acid addition salts, solvates and hydrates thereof.

2. Compounds according to claim 1,
R denotes a group selected from among
cis-4-(methoxycarbonylamino)-cyclohexyl, trans-4-(methoxycarbonylamino)-cyclohexyl,
cis-4-(N-methoxycarbonyl-N-methyl-amino)-cyclohaxyl, trans-4-(N-methoxycorbonyl-N-methyl-amino)-cyclohexyl,
cis-4-(ethyloxycarbonylamino)-cyclohexyl, trans-4-(ethyloxycarbonylamino)-cyclohexy,
cis-4-(N-ethyloxycarbonyl-N-methyl-amino)-cyclohexyl, trans-4-(N-ethyloxycarbonyl-N-methyl-amino)-cyclohexyl,
cis-4-(tert.-butoxycarbonylamino)-cyclohexyl, trans-4-(tert.-butoxycarbonylamino)-cyclohexyl,
cis-4-(N-tert.-butoxycarbonyl-N-methyl-amino)-cyclohexyl, trans-4-(N-tert: butoxycarbonyl-N-methyl-amino)-cyclohexyl,
cis-4-(methoxyacetyl-amino)-cyclohexyl, trans-4-(methoxyacetyl-amino)-cyclohexyl,
cis-4-(N-methoxyacetyl-N-methyl-amino)-cyclohexyl, trans-4-(N-methoxyacetyl-N-methyl-amino)-cyclohexyl,
cis-4-(dimethylaminocarbonyl-amino)-cyclohexyl, trans-4-(dimethylaminocarbonyl-amino)-cyclohexyl,
cis-4-(N-dimethylaminocarbonyl-N-methyl-amino)-cyclohexyl, trans-4-(N-dimethylaminocarbonyl-N-methyl-amino)-cyclohexyl,
cis-4-(morpholinocarbonyl-amino)-cyclohexyl, trans-4-(morpholinocarbonyl-amino)-cyclohexyl,
cis-4-(N-marpholinocarbonyl-N-methyl-amino)-cyclohexyl, trans-4-(N-morpholinocarbonyl-N-methyl-amino)-cyclohexyl,
cis-4-(piperazin-1-ylcarbonyl-amino)-cyclohexyl, trans-4-(piperazin-1-ylcarbonyl-amino)-cyclohexyl,
cis-4-(N-piperazin-1-ylcarbonyl-N-methyl-amino)-cyclahexyl, trans-4-(N-piperazin-1-ylcarbonyl-N-methyl-amino)-cyclohexyl,
cis-4-[(4-methyl-piperazin-1-ylcarbonyl)-amino]-cyclohexyl, trans-4-[(4-methyl-piperazin-1-ylcarbonyl)-amino]-cyclohexyl,
cis-4-[N-(4-methyl-piperazin-1-ylcarbonyl)-N-methyl-amino]-cyclohexyl, trans-4-[N-(4-methyl-piperazin-1-ylcarbonyl)-N-methyl-amino]-cyclohexyl,
cis-4-(methanesulphonylamino)-cyclohexyl, trans-4-(methanesulphonylamino)-cyclohexyl,
cis-4-(N-methanesulphonyl-N-methyl-amino)-cyclohexyl, trans-4-(N-methanesulphanyl-N-methyl-amino)-cyclohexyl, cis-4-phthalimido-cyclohexyl and trans-4-phthalimido-cyclohexyl,
optionally in the form of the tautomers and mixtures thereof, and optionally the pharmacologically acceptable acid addition salts, solvates and hydrates thereof.

3. Physiologically acceptable salts of the compounds according to claim 1 with inorganic or organic acids.

4. Medicament, containing a compound according to claim 1 or a physiologically acceptable salt according to claim 2 optionally together with one or more inert carriers and/or diluents.

5. Use of a compound according to at least one of claims 1 to 2 for preparing a medicament that is suitable for the treatment of benign or malignant tumours, for the prevention and treatment of diseases of the airways and lung as well as for the treatment of diseases of the gastro-intestinal tract and the bile duct and gall bladder.

6. Process for preparing the compounds of general formula I according to claim 1, **characterised in that**
a) a compound of formula (II) 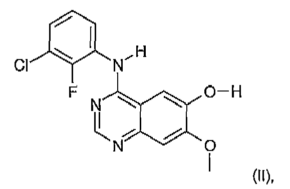 is reacted with a compound of general formula (III)
Z¹ - R (III),
wherein,
R is defined as in claim 1, and Z¹ denotes a leaving group or hydroxy group, or
b) a compound of general formula (IV) 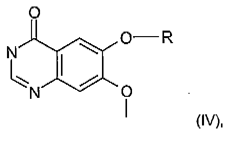 wherein R is defined as in claim 1,
is reacted with a halogenating agent to form an intermediate compound of general formula (V), 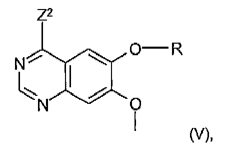 wherein R is defined as in claim 1, and Z² denotes a halogen atom, and is then reacted with 3-chloro-2-fluoro-aniline.

7. Process for preparing the compounds of general formula (I) according to claim 1, **characterised in that**
R denotes a group selected from among cis-4-(methoxycarbonylamino)-cyclohexyl, trans-4-(methoxycarbonylamino)-cyclohexyl,
cis-4-(N-methoxycarbonyl-N-methyl-amino)-cyclohexyl, trans-4-(N-methoxycarbonyl-N-methyl-amino)-cyclohexyl, cis-4-(ethyloxycarbonylamino)-cycichexyl, trans-4-(ethyloxycarbonylamino)-cyclohexyl, cis-4-(N-ethyloxycarbonyl-N-methyl-amino)-cyclohexyl, trans-4-(N-ethyloxycarbonyl-N-methyl-amino)-cyclohexyl, cis-4-(tert.-butoxycarbonylamino)-cyclohexyl, trans-4-(tert.-butoxycarbonylamino)-cyclohexyl, cis-4-(N-tert.-butoxycarbonyl-N-methyl-amino)-cyclohexyl, trans-4-(N-tert.-butoxycarbonyl-N-methyl-amino)-cyclohexyl,
cis-4-(acetylamino)-cyclohexyl, trans-4-(acetylamino)-cyclohexyl,cis-4-(N-acetyl-N-methyl-amino)-cyclohexyl, trans-4-(N-acetyl-N-methyl-amino)-cyclohexyl, cis-4-(methoxyacetyl-amino)-cyclohexyl, trans-4-(methoxyacetyl-amino)-cyclohexyl,cis-4-(N-methoxyacetyl-N-methyl-amino)-cyclohexyl, trans-4-(N-methoxyacetyl-N-methyl-amino)-cyclohexyl, cis-4-(dimethylaminocarbonyl-amino)-cyclohexyl, trans-4-(dimethylaminocarbonyl-amino)-cyclohexyl,cis-4-(N-dimethylaminocarbonyl-N-methyl-amino)-cyclohexyl, trans-4-(N-dimethylaminocarbonyl-N-methyl-amino)-cyclohexyl, cis-4-(morpholinocarbonyl-amino)-cyclohexyl, trans-4-(morpholinocarbonyl-amino)-cyclohexyl,cis-4-(N-morpholinocarbonyl-N-methyl-amino)-cyclohexyl, trans-4-(N-morpholinocarbonyl-N-methyl-amino )-cyclohexyl, cis-4-(piperazin-1-ylcarbonyl-amino)-cyclohexyl, trans-4-(piperazin-1-ylcarbonyl-amino)-cyclohexyl,
cis-4-(N-piperazin-1-ylcarbonyl-N-methyl-amino )-cyclohexyl, trans-4-(N-piperazin-1-ylcarbonyl-N-methyl-amino)-cyclohexyl, cis-4-[(4-methyl-piperazin-1-ylcarbonyl)-amino]-cyclohexyl, trans-4-[(4-methyl-piperazin-1-ylcarbonyl)-amino]-cyclohexyl, cis-4-[N-(4-methyl-piperazin-1-ylcarbonyl)-N-methyl-amino]-cyclohexyl, trans-4-[N-(4-methyl-piperazin-1-ylcarbonyl)-N-methyl-amino]-cyclohexyl, cis-4-(methanesulphonylamino)-cyclohexyl, trans-4-(methanesulphvnylamino)-cyclohexyl, cis-4-(N-methanesulphonyl-N-methyl-amino)-cyclohexyl and trans-4-(N-methanesulphonyl-N-methyl-amino)-cyclohaxyl,
a compound of general formula (VI) 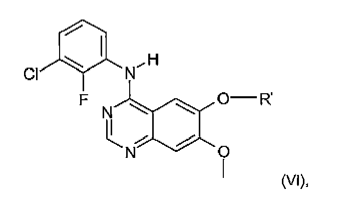 wherein
R' denotes a group selected from among cis-4-amino-cyclohex-1-yl, trans-4-amino-cyclohex-1-yl, cis-4-(methylamino)-cyclohex-1-yl and trans-4-(methylamino)-cyclohex-1-yl,
is reacted with a corresponding acylating agent.

8. Process for preparing the compounds of general formula (I) according to claim 1, **characterised in that**
R denotes a cis-4-phthalimido-cyclohex-1-yl or trans-4-phthalimido-cyclohex-1- yl group, and
a compound of general formula (VII) 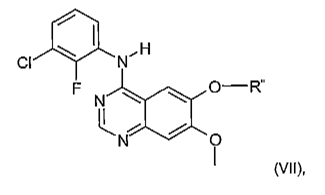 wherein
R" denotes a cis-4-amino-cyclohex-1-yl or trans-4-amino-cyclohex-1-yl group,
is reacted with phthalic anhydride or another reactive derivative of phthalic acid.

9. Medicament combinations which contain in addition to one or more compounds of formula 1 according to one of claims 1 or 2, as a further active substance, one or more compounds selected from among the categories of the betamimetics, anticholinergics, corticosteroids, other PDE4-inhibitors, LTD4-antagonists, EGFR-inhibitors, dopamine agonists, H1-antihistaminas, PAF-antagonists, PI3-kinase inhibitors, MPR4-inhibitors, iNOS-inhibitors and SYK-inhibitors or double or triple combinations thereof.

## Revendications

1. Hétérocycles bicycliques de la formule générale (I) : 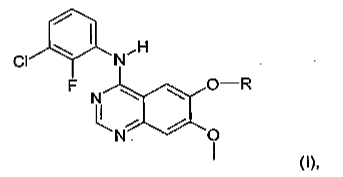 où
R représente un reste choisi parmi le groupe consistant en cis-4-aminocyclohexyle, trans-4-aminocyclohexyle, cis-4-méthylaminocyclohexyle, trans-4-méthylaminocyclohexyle, cis-4-(méthoxy-carbonylamino)cyclohexyle, trans-4-(méthoxy-carbonylamino)cyclohexyle, cis-4-(N-méthoxy-carbonyl-N-méthylamino)cyclohexyle, trans-4-(N-méthoxycarbonyl-N-méthylamino)cyclohexyle, cis-4-(éthoxycarbonylamino)cyclohexyle, trans-4-(éthoxy-carbonylamino)cyclohexyle, cis-4-(N-éthoxy-carbonyl-N-méthylamino)cyclohexyle, trans-4-(N-éthoxycarbonyl-N-méthylamino)cyclohexyle, cis-4-(butoxycarbonylamino)cyclohexyle, trans-4-(butoxycarbonylamino)cyclohexyle, cis-4-(N-butoxycarbonyl-N-méthylamino)-cyclohexyle, trans-4-(N-butoxycarbonyl-N-méthylamino)cyclohexyle, cis-4-(acétylamino)cyclohexyle, trans-4-(acétyl-amino)cyclohexyle, cis-4-(N-acétyl-N-méthylamino)-cyclohexyle, trans-4-(N-acétyl-N-méthylamino)cyclohexyle, cis-4-(méthoxyacétylamino)cyclohexyle, trans-4-(méthoxyacétylamino)cyclohexyle, cis-4-(N-méthoxyacétyl-N-méthylamino)cyclohexyle, trans-4-(N-méthoxyacétyl-N-méthylamino)cyclohexyle, cis-4-(diméthylaminocarbonylamino)cyclohexyle, trans-4-(diméthylaminocarbonylamino)-cyclohexyle, cis-4-(N-diméthylaminocarbonyl-N-méthylamino)cyclohexyle, trans-4-(N-diméthyl-aminocarbonyl-N-méthylamino)cyclohexyle, cis-4-(morpholinocarbonylamino)cyclohexyle, trans-4-(morpholinocarbonylamino)cyclohexyle, cis-4-(N-morpholinocarbonyl-N-méthylamino)cyclohexyle, trans-4-(N-morpholinocarbonyl-N-méthylamino)-cyclohexyle, cis-4-(pipérazin-1-ylcarbonylamino)-cyclohexyle, trans-4-(pipérazin-1-ylcarbonyl-amino)cyclohexyle, cis-4-(N-pipérazin-1-yl-carbonyl-N-méthylamino)cyclohexyle, trans-4-(N-pipérazin-1-ylcarbonyl-N-méthylamino)cyclohexyle, cis-4-[(4-méthylpipérazin-1-ylcarbonyl)amino]-cyclohexyle, trans-4-[(4-méthylpipérazin-1-yl-carbonyl)amino]cyclohexyle, cis-4-[N-(4-méthyl-pipérazin-1-ylcarbonyl)N-méthylamino]cyclohexyle, trans-4-[N-(4-méthylpipérazin-1-ylcarbonyl)N-méthylamino]cyclohexyle, cis-4-(méthanesulfonyl-amino)cyclohexyle, trans-4-(méthanesulfonyl-amino)cyclohexyle, cis-4-(N-méthanesulfonyl-N-méthylamino)cyclohexyle, trans-4-(N-méthane-sulfonyl-N-méthylamino)cyclohexyle, cis-4-phtalimidocyclohexyle, et trans-4-phtalimido-cyclohexyle,
le cas échéant sous forme de leurs tautomères et leurs mélanges, ainsi que le cas échéant, leurs sels d'addition d'acide, solvates et hydrates pharmacologiquement sans risque.

2. Composés selon la revendication 1, dans lesquels
R représente un reste choisi parmi le groupe consistant en cis-4-(méthoxycarbonylamino)cyclohexyle, trans-4-(méthoxycarbonylamino)cyclohexyle, cis-4-(N-méthoxycarbonyl-N-méthylamino)cyclohexyle, trans-4-(N-méthoxycarbonyl-N-méthyl-amino)-cyclohexyle, cis-4-(éthoxycarbonylamino)-cyclohexyle, trans-4-(éthoxycarbonylamino)cyclohexyle, cis-4-(N-éthoxycarbonyl-N-méthylamino)-cyclohexyle, trans-4-(N-éthoxycarbonyl-N-méthyl-amino)cyclohexyle, cis-4-(butoxycarbonylamino)-cyclohexyle, trans-4-(butoxycarbonylamino)cyclohexyle, cis-4-(N-butoxycarbonyl-N-méthylamino)-cyclohexyle, trans-4-(N-butoxycarbonyl-N-méthyl-amino)cyclohexyle, cis-4-(méthoxyacétylamino)-cyclohexyle, trans-4-(méthoxyacétylamino)cyclohexyle, cis-4-(N-méthoxyacétyl-N-méthylamino) - cyclohexyle, trans-4-(N-méthoxyacétyl-N-méthyl-amino)cyclohexyle, cis-4-(diméthylaminocarbonyl-amino)cyclohexyle, trans-4-(diméthylamino-carbonylamino)cyclohexyle, cis-4-(N-diméthyl-aminocarbonyl-N-méthylamino)cyclohexyle, trans-4-(N-diméthylaminocarbonyl-N-méthylamino)cyclohexyle, cis-4-(morpholinocarbonylamino)cyclohexyle, trans-4-(morpholinocarbonylamino)cyclohexyle, cis-4-(N-morpholinocarbonyl-N-méthylamino)cyclohexyle, trans-4-(N-morpholinocarbonyl-N-méthylamino)-cyclohexyle, cis-4-(pipérazin-1-ylcarbonylamino)-cyclohexyle, trans-4-(pipérazin-1-ylcarbonyl-amino)cyclohexyle, cis-4-(N-pipérazin-1-yl-carbonyl-N-méthylamino)cyclohexyle, trans-4-(N-pipérazin-1-ylcarbonyl-N-méthylamino)cyclohexyle, cis-4-[(4-méthylpipérazin-1-ylcarbonyl)amino]-cyclohexyle, trans-4-[(4-méthylpipérazin-1-yl-carbonyl)amino]cyclohexyle, cis-4-[N-(4-méthyl-pipérazin-1-ylcarbonyl)-N-méthylamino]cyclohexyle, trans-4-[N-(4-méthylpipérazin-1-ylcarbonyl)-N-méthylamino]cyclohexyle, cis-4-(méthanesulfonyl-amino)cyclohexyle, trans-4-(méthanesulfonyl-amino)cyclohexyle, cis-4-(N-méthanesulfonyl-N-méthylamino)cyclohexyle, trans-4-(N-méthane-sulfonyl-N-méthylamino)cyclohexyle, cis-4-phtalimidocyclohexyle, et trans-4-phtalimido-cyclohexyle,
le cas échéant sous forme de leurs tautomères et leurs mélanges, ainsi que le cas échéant, leurs sels d'addition d'acide, solvates et hydrates pharmacologiquement sans risque.

3. Sels physiologiquement compatibles des composés selon la revendication 1, avec des acides inorganiques ou organiques.

4. Agent pharmaceutique contenant un composé selon la revendication 1 ou un sel physiologiquement compatible selon la revendication 2, ainsi que le cas échéant, un ou plusieurs supports et/ou agents de dilution inertes.

5. Utilisation d'un composé selon au moins l'une des revendications 1 et 2, pour la préparation d'un agent pharmaceutique, qui est approprié pour le traitement de tumeurs bénignes ou malignes, pour la prévention et le traitement de maladies des voies respiratoires et des poumons ainsi que pour le traitement de maladies du tractus gastro-intestinal et du canal cholédoque et de la vésicule biliaire.

6. Procédé de préparation des composés de la formule générale I selon la revendication 1, **caractérisé en ce que**
a) on fait réagir un composé de formule (II) : 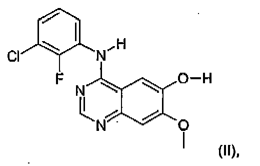 avec un composé de formule générale (III) :
Z¹-R (III)
où R est tel que défini à la revendication 1, et Z¹ représente un groupe partant ou un radical hydroxyle, ou
b) on fait réagir un composé de formule générale (IV) : 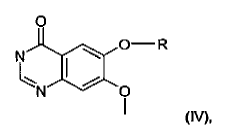 où R est tel que défini à la revendication 1,
avec un agent d'halogénation, pour donner un composé intermédiaire de la formule générale (V) : 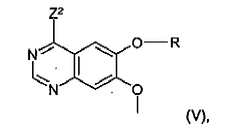 où R est tel que défini à la revendication 1, et
Z² représente un atome d'halogène, et ensuite on fait réagir avec la 3-chloro-2-fluoroaniline.

7. Procédé de préparation de composés de la formule générale (I) selon la revendication 1, **caractérisé en ce que** R est un reste choisi parmi le groupe consistant en cis-4-(méthoxycarbonyl-amino)cyclohexyle, trans-4-(méthoxycarbonylamino)-cyclohexyle, cis-4-(N-méthoxycarbonyl-N-méthyl-amino)cyclohexyle, trans-4-(N-méthoxycarbonyl-N-méthylamino)cyclohexyle, cis-4-(éthoxycarbonyl-amino)cyclohexyle, trans-4-(éthoxycarbonylamino)-cyclohexyle, cis-4-(N-éthoxycarbonyl-N-méthyl-amino)cyclohexyle, trans-4-(N-éthoxycarbonyl-N-méthylamino)cyclohexyle, cis-4-(butoxycarbonyl-amino)cyclohexyle, trans-4-(butoxycarbonylamino)-cyclohexyle, cis-4-(N-butoxycarbonyl-N-méthyl-amino)cyclohexyle, trans-4-(N-butoxycarbonyl-N-méthylamino)cyclohexyle, cis-4-(acétylamino)-cyclohexyle, trans-4-(acétylamino)cyclohexyle, cis-4-(N-acétyl-N-méthylamino)cyclohexyle, trans-4-(N-acétyl-N-méthylamino)cyclohexyle, cis-4-(méthoxyacétylamino)cyclohexyle, trans-4-(méthoxyacétylamino)cyclohexyle, cis-4-(N-méthoxy-acétyl-N-méthylamino)cyclohexyle, trans-4-(N-méthoxyacétyl-N-méthylamino)cyclohexyle, cis-4-(diméthylaminocarbonylamino]cyclohexyle, trans-4-(diméthylaminocarbonylamino)cyclohexyle, cis-4-(N-diméthylaminocarbonyl-N-méthylamino)cyclohexyle, trans-4-(N-diméthylaminocarbonyl-N-méthylamino)cyclohexyle, cis-4-(morpholinocarbonyl-amino)cyclohexyle, trans-4-(morpholinocarbonyl-amino)cyclohexyle, cis-4-(N-morpholinocarbonyl-N-méthylamino)cyclohexyle, trans-4-(N-morpholino-carbonyl-N-méthylamino)cyclohexyle, cis-4-(pipérazin-1-ylcarbonylamino)cyclohexyle, trans-4-(pipérazin-1-ylcarbonylamino)cyclohexyle, cis-4-(N-pipérazin-1-ylcarbonyl-N-méthylamino)cyclohexyle, trans-4-(N-pipérazin-1-ylcarbonyl-N-méthylamino)cyclohexyle, cis-4-[(4-méthyl-pipérazin-1-ylcarbonyl)amino]cyclohexyle, trans-4-[(4-méthylpipérazin-1-ylcarbonyl)amino]cyclohexyle, cis-4-[N-(4-méthylpipérazin-1-yl-caxbonyl)-N-méthylamino]cyclohexyle, trans-4-[N-(4-méthylpipérazin-1-ylcarbonyl)-N-méthylamino]-cyclohexyle, cis-4-(méthanesulfonylamino)cyclohexyle, trans-4-(méthanesulfonylamino)cyclohexyle, cis-4-(N-méthanesulfonyl-N-méthylamino)cyclohexyle, et trans-4-(N-méthanesulfonyl-N-méthyl-amino)cyclohexyle,
et **en ce que** l'on fait réagir un composé de la formule générale (VI) :
où 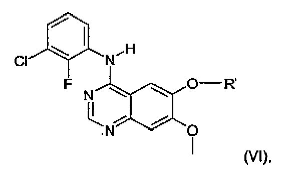 R' représente un reste choisi parmi le groupe consistant en cis-4-aminocyclohex-1-yle, trans-4-aminocyclohex-1-yle, cis-4-(méthylamino)cyclohex-1-yle, et trans-4-(méthylamino)cyclohex-1-yle,
avec un agent d'acylation approprié.

8. Procédé de préparation de composés de la formule générale (I) selon la revendication 1, **caractérisé en ce que** R représente un groupe cis-4-phtalimidocyclohex-1-yle ou trans-4-phtalimidocyclohex-1-yle,
et **en ce que** l'on fait réagir un composé de la formule générale (VII) :
où 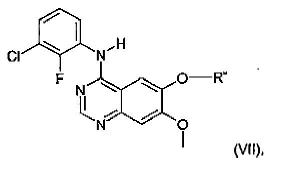 R" représente un groupe cis-4-aminocyclohex-1-yle ou trans-4-aminocyclohex-1-yle,
avec l'anhydride phtalique ou un autre dérivé réactif de l'acide phtalique.

9. Combinaisons d'agents pharmaceutiques, qui contiennent outre un ou plusieurs composés de formule I selon l'une des revendications 1 ou 2, comme autre agent actif, un ou plusieurs composés qui sont choisis parmi les classes des bêta-mimétiques, des anti-cholinergiques, des corticostéroïdes, d'autres inhibiteurs de PDE-4, des antagonistes de LTD-4, des inhibiteurs de EGFR, des agonistes de la dopamine, des antihistaminiques H1, des antagonistes de PAF, des inhibiteurs de la kinase PI3, des inhibiteurs de MPR4, des inhibiteurs de iNOS et des inhibiteurs de SYK ou des combinaisons doubles ou triples de ceux-ci.
